# EUROPEAN PATENT APPLICATION

(11) **EP 4 733 308 A1**
(43) Date of publication of application: **29.04.2026**
(21) Application number: 24825312.2
(22) Date of filing: 20.06.2024
(51) Int. Cl.: C07K 5/097, C07K 5/117, C07K 5/078, C07K 5/033, A61K 51/08, A61K 51/04, A61P 35/00, C07K 1/06, C07K 1/13, A61K 103/00

(54) **FAP-TARGETING COMPOUND, AND RADIONUCLIDE-LABELED COMPLEX BASED THEREON, AND PREPARATION METHOD THEREFOR AND USE THEREOF**

(30) Priority: 21.06.2023 CN 202310747044
(71) Applicant: Shanghai Institute of Materia Medica, Chinese Academy of Sciences, Shanghai 201203 (CN)
(72) Inventor: CHENG, Zhen, Shanghai 201203 (CN); QU, Chunrong, Shanghai 201203 (CN); LAI, Chaoquan, Shanghai 201203 (CN); CAO, Rui, Shanghai 201203 (CN)
(74) Representative: Petraz, Gilberto Luigi
(86) International application number: PCT/CN2024/100415
(87) International publication number: WO 2024/260425

(57) **Abstract**

Disclosed in the present invention are a FAP-targeting compound, and a radionuclide-labeled complex based thereon and a preparation method therefor and the use thereof. The FAP-targeting compound has a structure as shown in formula (I), and has high affinity for a FAP target. A radiopharmaceutical obtained by means of labeling the FAP-targeting compound with a radionuclide such as ⁶⁸Ga shows higher tumor uptake and longer tumor retention time, and can be used for the application and further development of FAP-targeting drugs.

## Description

### TECHNICAL FIELD

The present invention belongs to the field of biomedicine, and specifically relates to a fibroblast activation protein (FAP)-targeting compound of formula (I), a radionuclide-labeled complex based thereon, a method for preparing the same, and their use in preparation of a medicament for diagnosis, prevention and/or treatment of a disease characterized by high FAP expression.

### BACKGROUND

90% of deaths in patients with cancers are caused by tumor cell metastasis, yet the lack of preventative and therapeutic strategies for tumor metastasis remain a problem. A tumor is a complex composed of tumor cells, surrounding stromal cells, and non-cellular components. Tumor development is a dynamic process of mutual promotion and co-evolution between tumor cells and their microenvironment. The tumor microenvironment (TME) includes cells and the extracellular matrix (ECM). Its formed components are primarily cellular components, including immune cells, endothelial cells, and fibroblasts. Among these, cancer associated fibroblasts (CAFs) are the most abundant stromal cells in the tumor microenvironment, accounting for approximately 50% of the total number of cells in tumor tissue. CAFs play a crucial role in tumor growth, metastasis, drug resistance, and treatment resistance, making them a hot topic in cancer diagnosis and treatment research.

In cancer, fibroblast activation protein (FAP) has become a unique marker of tumor-associated fibroblasts (CAFs) and a key regulator and driver of the tumor microenvironment (TME). CAF is one of the largest components of the TME, promoting tumor growth and cell invasion by secreting pro-inflammatory factors and growth factors and remodeling the ECM. Besides cells within the TME, FAP expression can also be found in malignant epithelial cells. FAP promotes tumor growth through ECM remodeling, leading to the formation of active cancer matrix, which is essential for cancer cell invasion and metastasis. Increased FAP expression in the pre-invasive portion of colorectal cancer tumor samples has been observed clinically, which further supports its role in invasion and metastasis. FAP also promotes the formation of an immunosuppressive TME by activating tumor-promoting inflammation. FAP is selectively expressed on the surface of stromal fibroblasts in more than 90% of epithelial malignancies, including breast cancer, ovarian cancer, lung cancer, colorectal cancer, gastric cancer, pancreatic cancer, and skin melanoma. FAP is usually not expressed in benign and precancerous epithelial tumors, such as colorectal adenomas, phyllodes tumors of the breast, and fibroadenomas. FAP is generally not expressed in normal human tissues, only present in the cervix and endometrium, and is transiently expressed during embryonic development. Because FAP plays a crucial role in TME, there is growing interest in using it as a target for imaging and therapy.

In recent years, many FAP inhibitors have entered clinical trials, such as ⁶⁸Ga-FAPI-04, ⁶⁸Ga-FAPI-46, ⁶⁸Ga-oncoFAP-DOTAGA, and ¹⁷⁷Lu-FAPI-04, providing powerful tools for precise localization and targeted killing of various cancer lesions. However, their relatively short retention time in tumors, low absolute tumor uptake, and rapid in vivo clearance result in unsatisfactory uptake and imaging effects in tumors with low FAP expression, as well as limited killing effect of long-half-life therapeutic radionuclides on target cells or tissues. Therefore, it is of great significance to develop FAP-targeting probes with higher tumor uptake, longer retention time, low uptake in non-target tissues, and rapid clearance for advancing integrated FAP-targeted diagnosis and treatment.

### SUMMARY OF THE INVENTION

FAP is selectively and highly expressed in over 90% of epithelial malignancies, making it an ideal target for developing tumor-targeting probes. Considering the low uptake and short retention time of existing FAP-targeting small molecule compounds in tumors, the present invention provides a compound of formula (I) that can simultaneously recognize different amino acid sites of FAP to increase affinity and tumor uptake, thus exhibiting high affinity for FAP and can be used for FAP targeting. Furthermore, the inventor has found that radionuclide-labeled complexes based on the compound can specifically recognize FAP targets and increase tumor uptake, thereby improving tumor detection rate and/or therapeutic efficacy.

On such basis, one object of the present invention is to provide a compound of formula (I), or a pharmaceutically acceptable salt, enantiomer, diastereomer, racemate, transisomer, polymorph, solvate, or isotopically labeled compound thereof.

Another object of the present invention is to provide a method for preparing the compound of formula (I).

Another object of the present invention is to provide a radionuclide-labeled complex, which is obtained by labeling the compound of formula (I) or a pharmaceutically acceptable salt, enantiomer, diastereomer, racemate, transisomer, polymorph, solvate, or isotopically labeled compound thereof as a ligand with a radionuclide.

Another object of the present invention is to provide a method for preparing the radionuclide-labeled complex.

Another object of the present invention is to provide a pharmaceutical composition comprising one or more selected from the compounds of formula (I), or a pharmaceutically acceptable salt, enantiomer, diastereomer, racemate, transisomer, polymorph, solvate, or isotopically labeled compound thereof, and the above radionuclide-labeled complex, and optionally a pharmaceutically acceptable excipient.

Another object of the present invention is to provide the use of the compound of formula (I) or a pharmaceutically acceptable salt, enantiomer, diastereomer, racemate, transisomer, polymorph, solvate, or isotopically labeled compound thereof, or the above radionuclide-labeled complex, in preparation of a reagent for inhibiting FAP activity.

A further object of the present invention is to provide the use of the compound of formula (I) or the pharmaceutically acceptable salt, enantiomer, diastereomer, racemate, transisomer, polymorph, solvate, or isotopically labeled compound thereof, or the above radionuclide-labeled complex, in the preparation of a medicament or reagent for diagnosing, preventing, and/or treating a disease characterized by high FAP expression.

To achieve the above objects, the present invention provides the following technical solutions.

In a first aspect, provided is a compound of formula (I), or a pharmaceutically acceptable salt, enantiomer, diastereomer, racemate, transisomer, polymorph, solvate, or isotopically labeled compound thereof, 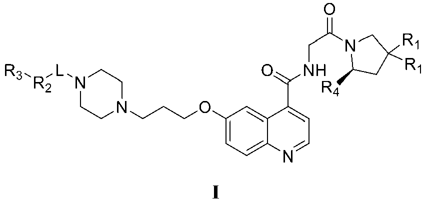
wherein, each of R₁s is independently a halogen or H, preferably F;
R₂ is -(W₁)ₙ₁-(W₁)ₙ₂-(W₁)ₙ₃-(W₁)ₙ₄-(W₁)ₙ₅-(W₁)ₙ₆-, where n1 to n6 are each independently 0 or 1;
each of W₁s is independently selected from 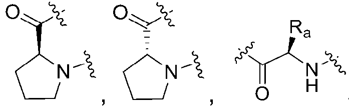 and 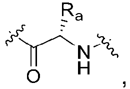 where Rₐ is selected from H, C1-C3 alkyl (e.g., methyl, ethyl, n-propyl, isopropyl), and the W₁s are connected from each other via an amide bond formed by the connection of a carbonyl group with N, provided that three or more adjacent W₁s are not simultaneously 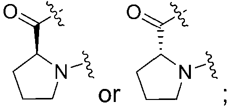
R₃ is a bifunctional chelator, a fluorescent reporter group, or a therapeutic drug;
L is -(CH₂)ₙ₇-, where n7 is an integer from 0 to 40; each CH₂ is independently and optionally replaced by -O-, -NH-, -(CO)-, -NH(CO)-, or -(CO)-NH-, provided that no two adjacent CH₂ groups are replaced simultaneously;
R₄ is cyano (-CN) or borate (-B(OH)₂).

The bifunctional chelator is a chelator that simultaneously has a function of chelating a radionuclide and a function of connecting to a targeted molecular probe, such as 1,4,7,10-tetraazacyclododecane-1,4,7,10-tetraacetic acid (DOTA), triethylenetetramine (TETA), 2-(4,7-biscarboxymethyl[1,4,7]triazacyclononyl-1-yl-ethyl)carbonyl-methylamino] acetic acid (NETA), diethylenetriamine-N,N,N',N',N"-pentaacetic acid (DTPA), N,N-di(2-hydroxyphenyl)ethylenediamine-N,N'-diacetic acid (HBED), and 2,2',",2‴-(5²,13²-dihydroxy-5⁵,13⁵-dimethyl-3,7,11,15-tetraaza-1,9(2,6)-dipyridin-5,13(1,3)-dibenzocyclohexanedione-3,7,1,11,15-tetrayl)tetraacetic acid (DAR); preferably 1,4,7,10-tetraazacyclododecane-1,4,7,10-tetraacetic acid (DOTA).

The fluorescent reporter group is a group capable of absorbing and emitting light within a certain wavelength range and releasing light energy, including but not limited to visible light groups, near-infrared region I groups, and near-infrared region II groups.

The visible light group may be any one selected from fluorescein, rhodamine, fluorescein isothiocyanate, cyanine fluorescent dyes (e.g., Cy2, etc.), green fluorescent protein, quantum dots, nanoparticles, F16, etc.

The near-infrared region I group may be any one selected from cyanine dyes (e.g., Cy3, Cy3.5, Cy5, Cy5.5, Cy7, Cy7.5, etc.), BODIPY dyes (e.g., fluoroboron dipyrrole, azafluoroboron dipyrrole, etc.), rhodamine dyes (e.g., rhodamine green, rhodamine 6G, tetramethylrhodamine, rhodamine B, lissamine rhodamine, X-rhodamine, Texas red, silyl rhodamine, etc.), quantum dots, nanoparticles, phthalocyanine, etc.

The near-infrared region II group may be any one selected from cyanine dyes (e.g., Cy7, Cy7.5, etc.), D-A-D dyes (e.g., CH-1055, CH-4T, FT-TQT, etc.), BODIPY dyes (e.g., NJ960, NJ1030, NJ1060, PCP-BDP2, etc.), quantum dots, and nanoparticles.

The therapeutic drug includes small molecule inhibitors, monoclonal antibody drugs, bioalkylating agents, cytotoxic drugs, hormonal drugs, and biological response modifiers, etc.

In some embodiments, each of the R₁s is H or F.

In some embodiments, R₂ is selected from 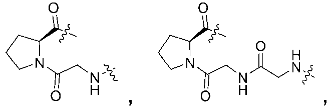 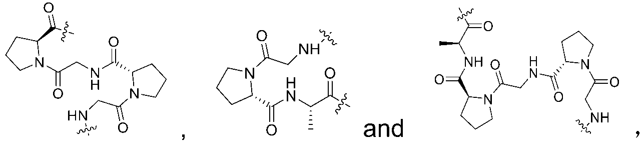 and preferably is

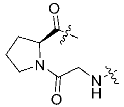

In some embodiments, R₃ is selected from: 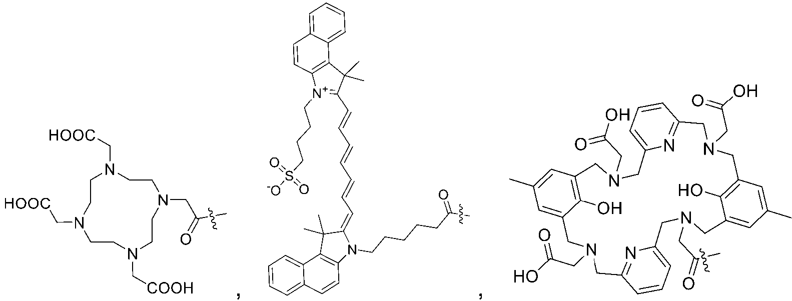 and 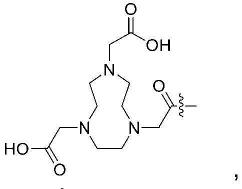 and preferably is 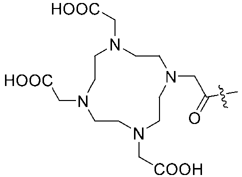 or

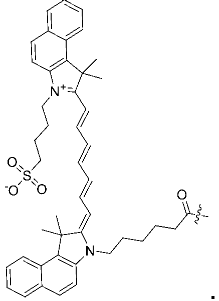

In some embodiments, L is -(CH₂)ₙ₇-; n7 is an integer from 0 to 30, more preferably from 0 to 12, and even more preferably is 0, 3, or 10; wherein each -CH₂- is independently and optionally replaced by -O-, -NH-, or -(CO)-, provided that no two adjacent -CH₂- groups are replaced.

In some embodiments, L is absent, or selected from -NH-(CH₂)ₙ₈-(CO)-, -NH-(CH₂CH₂O)ₙ₉-(CH₂)ₙ₈-(CO)-, where n8 and n9 are each independently an integer from 1 to 10 (e.g., 1, 2, 3, 4, 5, 6, 7, 8, 9, 10), preferably from 1 to 8, from 1 to 6, or from 1 to 4.

In some embodiments, R₄ is cyano (-CN).

In some embodiments, the compound of formula (I) is selected from formulas (II-1) and (II-2): 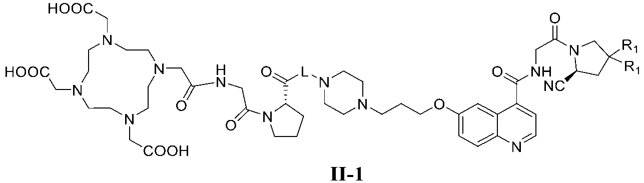 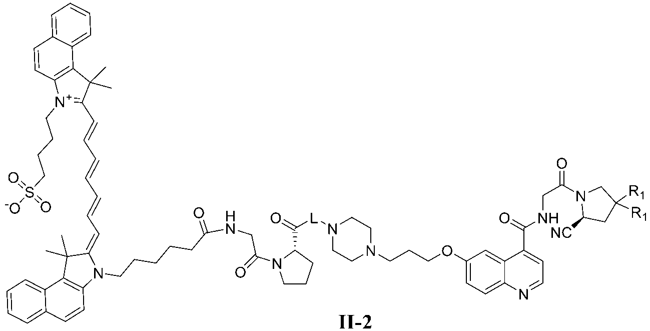 where R₁ and L are defined as above.

In some embodiments, the compound of formula (I) is selected from:

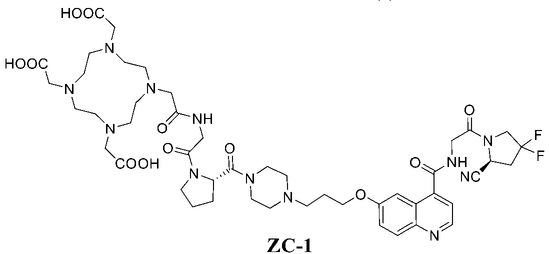

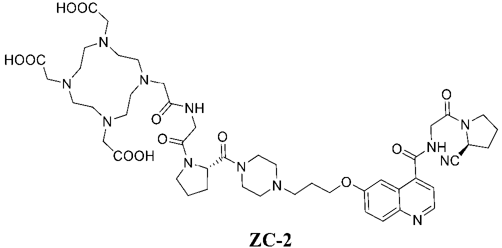

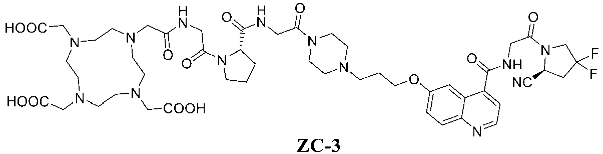

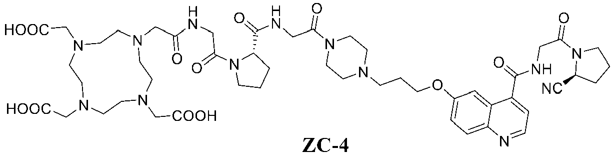

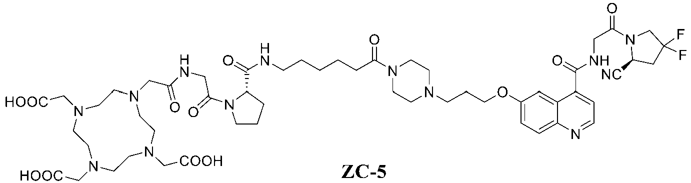

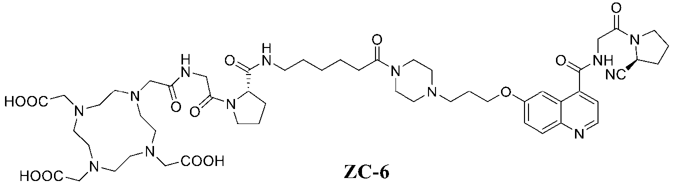

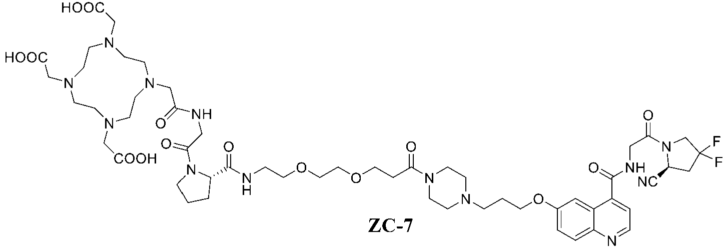

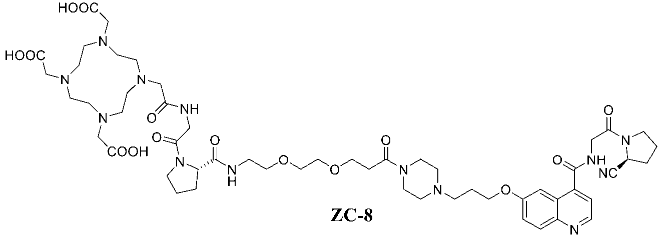

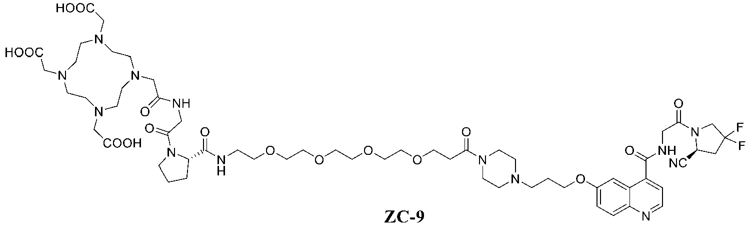

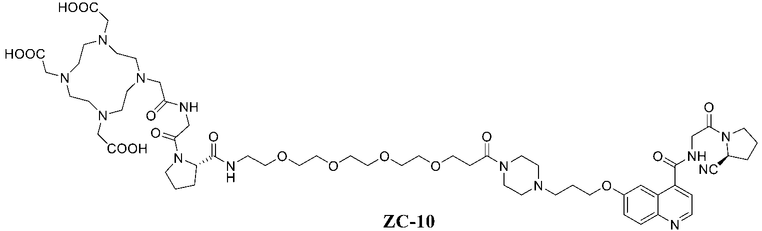

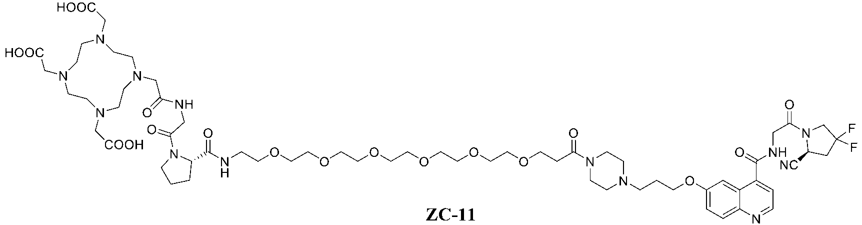

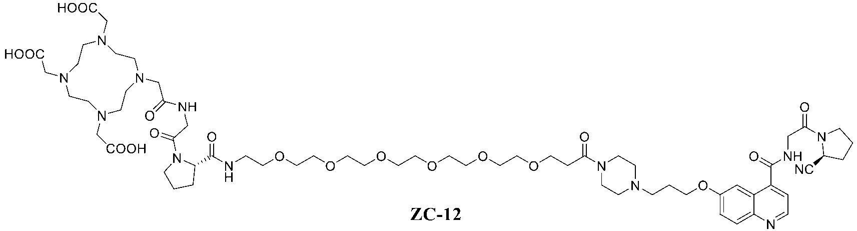

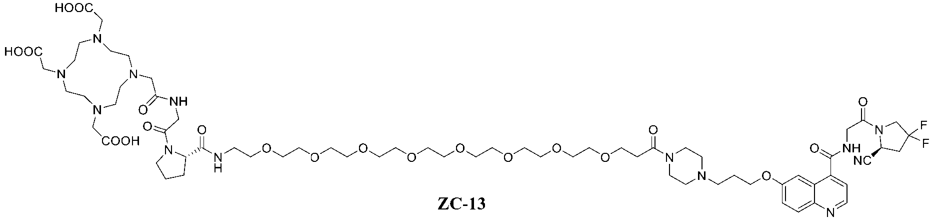

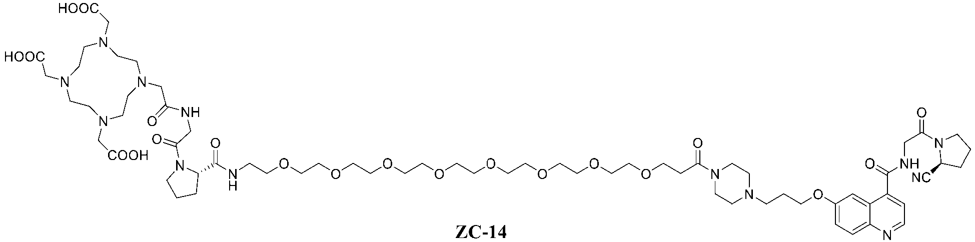

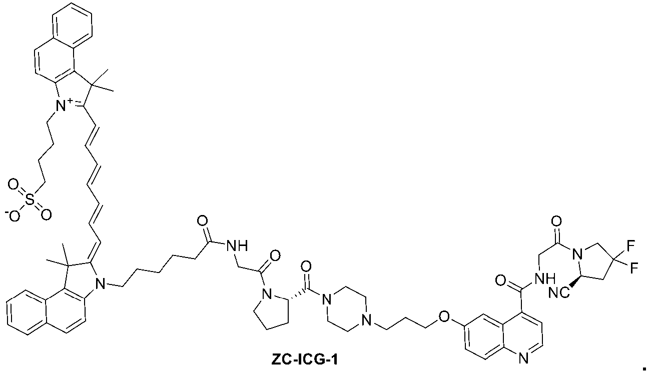

The present invention further provides a method for preparing compound ZC-1, comprising the steps of:
(1) demethylating compound 1 to obtain compound 2; wherein the reaction may be carried out in an aqueous solution of hydrogen bromide;
(2) esterifying compound 2 with methanol to obtain compound 3; wherein the reaction may be carried out in the presence of thionyl chloride;
(3) brominating compound 4 to generate compound 5; wherein the reaction may be carried out with carbon tetrabromide in the presence of triphenylphosphine;
(4) substituting compound 5 with compound 3 to generate compound 6; wherein the reaction may be carried out in the presence of potassium carbonate;
(5) ester-hydrolyzing compound 6 to generate compound 7; wherein the reaction may be carried out in the presence of LiOH;
(6) subjecting compound 7 to an amide condensation reaction with (S)-1-(2-aminoacetyl)-4,4-difluoropyrrolidine-2-carboxynitrile to generate compound 8;
(7) Boc-deprotecting compound 8 to generate compound 9 (this reaction may be carried out in the presence of trifluoroacetic acid), and then condensating compound 9 with Boc-gly-pro-OH to generate compound 10;
(8) Boc-deprotecting compound 10 (this reaction may be carried out under in the presence of trifluoroacetic acid), and then reacting with DOTA-NHS to obtain compound ZC-1.

The synthetic route of above specific steps is shown below:

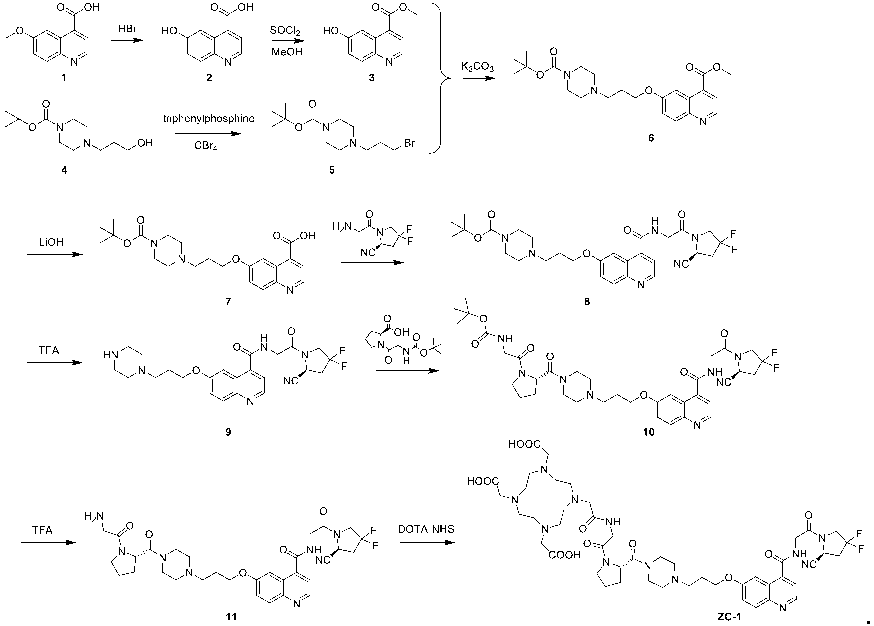

The other compounds in the present invention (e.g., compounds ZC-2 to ZC-14) may be prepared by a method similar to that for preparing compound ZC-1, for example, (S)-1-(2-aminoacetyl)-4,4-difluoropyrrolidine-2-carboxynitrile reacting with compound 7 may be replaced with (S)-1-(2-aminoacetyl)pyrrolidine-2-carboxynitrile, or compound 9 may be coupled with a carboxylic acid compound containing an L group and an L protecting group (e.g., BOC) (e.g., BOC glycine, 6-[(tert-butoxycarbonyl)amino]hexanoic acid, N-tert-butoxycarbonyl-diethylene glycol-carboxylic acid, 15-(Boc-amino)-4,7,10,13-tetraoxapentadecanoic acid, 21-(BOC-amino)-4,7,10,13,16,19-hexaoxadocosuccinic acid, etc.), deprotected the L protecting group, condensated with Boc-gly-pro-OH, deprotected the Boc protecting group and finally coupled with DOTA-NHS; or both of them are replaced.

In a second respect, the present invention further provides a radionuclide-labeled complex, which is obtained by labeling the compound of formula (I) or the pharmaceutically acceptable salt, enantiomer, diastereomer, racemate, transisomer, polymorph, solvate, or isotopically labeled compound thereof of the present invention as a ligand with a radionuclide M. The radionuclide-labeled complex may be used as a tumor radioactive diagnostic or therapeutic probe, that is, as a radionuclide diagnostic probe or a radionuclide therapeutic probe.

The radionuclide M includes radiodiagnostic nuclides and radiotherapeutic nuclides.

The radiodiagnostic nuclide may be, for example, any one selected from: ⁸⁶Y, ¹⁸F, ⁵¹Mn, ⁵²mMn, ^{52g}Mn, Al[¹⁸F], ⁶⁴Cu, ⁶⁷Ga, ⁶⁸Ga, ⁸⁹Zr, 99mTc, ¹¹¹In, 1231, 1241, ¹²⁵I, etc., preferably any one selected from: ⁸⁶Y, Al[¹⁸F], ⁶⁴Cu, ⁶⁸Ga, ⁸⁹Zr, ^{99m}Tc, ¹²⁴I, etc. The radiotherapeutic nuclide may be, for example, any one selected from: ⁶⁷Cu, ⁹⁰Y, ¹²⁵I, ¹³¹I, ¹⁵³Sm, ¹⁶⁶Ho, ¹⁷⁷Lu, ¹⁸⁶Re, ¹⁸⁸Re, ²¹¹At, ²¹²Pb, ²¹²Bi, ²¹³Bi, ²²³Ra, ²²⁵Ac, ²²⁷Th, etc.; preferably any one selected from: ⁶⁷Cu, ⁹⁰Y, ¹²⁵I, ¹³¹I, ¹⁷⁷Lu, ²²³Ra, ²²⁵Ac, ²¹¹At, etc.; more preferably ⁶⁸Ga, ¹⁷⁷Lu or ⁹⁰Y, etc.

**In** some embodiments, the radionuclide-labeled complex has a structure of formula (III): 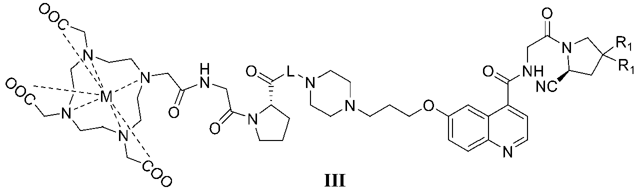 wherein,
L and R₁s are defined as above;
M is selected from the above radionuclides.

In some embodiments, M is any one selected from ⁶⁸Ga, ¹⁷⁷Lu, and ⁹⁰Y.

In some embodiments, the radionuclide-labeled complex has the structure of:

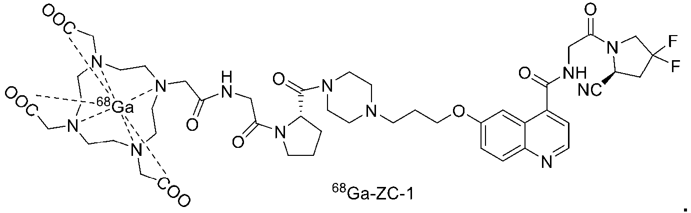

The radionuclide-labeled complex of the present invention may be prepared by various existing labeling methods using a compound containing a radionuclide and the compound of formula (I) of the present invention. Preferred labeling methods of the present invention may be, but not limited to, a wet method or a lyophilization method described below.

The wet labeling method comprises the steps of: dissolving an appropriate amount of the compound of formula (I) or the pharmaceutically acceptable salt, enantiomer, diastereomer, racemate, transisomer, polymorph, solvate, or isotopically labeled compound thereof of the present invention in a buffer solution or deionized water to obtain a solution; adding a radionuclide solution to the obtained solution and reacting under a sealed condition for 5-40 min to generate a radionuclide-labeled complex.

The lyophilization labeling method comprises the steps of: dissolving an appropriate amount of the compound of formula (I) or the pharmaceutically acceptable salt, enantiomer, diastereomer, racemate, transisomer, polymorph, solvate, or isotopically labeled compound thereof of the present invention in a buffer solution or deionized water to obtain a solution; after sterile filtration, dispensing the solution into a container, lyophilizing and sealing the container to obtain a lyophilized kit; adding an appropriate amount of an acetic acid solution or buffer solution to the lyophilized kit to dissolve the content, then adding a corresponding radionuclide solution, and reacting under a sealed condition for 5-40 min to generate a radionuclide-labeled complex. Among others, the container used for dispensing is preferably a cryovial or a tube vial for antibiotic use. Furthermore, an excipient, such as mannitol or ascorbic acid, etc., may be added to the kit depending on the shape of the lyophilized powder in the kit, and the optimal powder shape in the kit may be realized by adjusting the amounts of the compound of formula (I) of the present invention and the excipient.

The product obtained from the wet or lyophilized labeling methods may be further processed via conventional operations (such as chromatographic purification, solvent removal by rotary evaporation, dissolution of the residue with PBS or water or a physiological saline, sterile filtration, etc.) to prepare an injection solution.

In some embodiments, the method for preparing the radionuclide-labeled complex using the compound of formula (I) as a ligand is a wet labeling method, which comprises the steps of: dissolving the compound of formula (I) in a buffer solution or deionized water; adding a fresh radionuclide solution thereto, reacting at 37-90°C for 5-40 min under a sealed condition, and cooling; diluting the reaction solution with water and then purifying on a Sep-Pak C18 chromatographic column; rinsing the chromatographic column with a buffer or water to remove unreacted radioactive ions; rinsing with a hydrochloric acid ethanol solution or an ethanol solution; and then diluting with physiological saline or PBS and sterilely filtering the same to obtain an injection solution of a radionuclide-labeled complex having a structure of formula (III); wherein the radionuclide M is ⁶⁸Ga, ¹⁷⁷Lu, ⁹⁰Y,or the like.

In some embodiments, the method for preparing the radionuclide-labeled complexes using the compound of formula (I) as a ligand is a lyophilization labeling method, which comprises the steps of: dissolving the compound of formula (I) and other necessary reagents in a buffer solution; sterilely filtering the resulting solution and aliquoting into cryovials; lyophilizing and sealing the cryovials to obtain a lyophilized kit; adding an appropriate amount of a buffer solution to the lyophilized kit for dissolving, then adding a freshly prepared radionuclide solution; reacting at 37-120°C for 5-40 min under a sealed condition, and cooling; diluting the reaction solution with water and purifying on a Sep-Pak C18 chromatographic column; rinsing the chromatographic column with a buffer solution or water to remove unreacted radioactive ions; rinsing with a hydrochloric acid ethanol solution or an ethanol solution; and then diluting with a physiological saline or PBS and sterilely filtering the same to obtain an injection solution of a radionuclide-labeled complex having a structure of formula (III); wherein the radionuclide is ⁶⁸Ga, ¹⁷⁷Lu, ⁹⁰Y, or the like.

Other chemical substances used in the above synthesis steps are commercially available products.

The buffer solution is a substance that stabilizes the pH of the reaction solution and may be acetate, lactate, tartrate, malate, maleate, succinate, ascorbate, carbonate, and phosphate, as well as mixtures thereof, etc.

In another aspect, the present invention provides a pharmaceutical composition comprising one or more selected from the compound of formula (I), the pharmaceutically acceptable salt, enantiomer, diastereomer, racemic mixture, transisomer, a polymorph, solvate, and isotopically labeled compound thereof, or the above radionuclide-labeled complex; and optionally a pharmaceutically acceptable excipient.

In yet another aspect, the present invention provides use of the compound of formula (I) or the pharmaceutically acceptable salt, enantiomer, diastereomer, racemic mixture, transisomer, polymorph, solvate, or isotopically labeled compound thereof, or the above radionuclide-labeled complex, in preparation of a reagent for inhibiting FAP activity.

In further aspect, the present invention also provides use of the compound of formula (I) or the pharmaceutically acceptable salt, enantiomer, diastereomer, racemate, transisomer, polymorph, solvate, or isotopically labeled compound thereof, or the above radionuclide-labeled complex, in the preparation of a medicament or reagent for diagnosis, prevention, and/or treatment of a disease characterized by high FAP expression.

In some embodiments, the present invention provides use of the compound of formula (I) or the pharmaceutically acceptable salt thereof, or the above radionuclide-labeled complex, in preparation of a medicament or reagent for radionuclide therapy or imaging of a tumor with high FAP expression.

In the uses described in the present invention, the complex may be prepared into an injection for intravenous administration, but the present invention is not limited thereto.

In the uses described in the present invention, the tumor with high FAP expression includes, but is not limited to, breast cancer, ovarian cancer, lung cancer, colorectal cancer, gastric cancer, and pancreatic cancer.

### Beneficial effects

The compound of formula (I) and its radionuclide complex provided by the present invention can specifically target FAP and inhibit FAP activity. Biological test results revealed that they has significantly prolonged blood circulation half-life, enhanced tumor uptake and enrichment, and prolonged retention time, which are not found in other existing FAPI imaging agents (such as FAPI-04), and are thus suitable for radionuclide therapy and imaging of a tumor with high FAP expression.

### DESCRIPTION OF THE DRAWINGS

Fig. 1 shows a HPLC chromatogram of compound ZC-1 prepared in Example 1.
Fig. 2 shows a simulated docking binding diagram of compounds ZC-1, FAPI-04, with FAP in Test example 1.
Fig. 3 shows in vitro stability diagram of ⁶⁸Ga-ZC-1 at 37°C at different time points in Test example 3.
Fig. 4 shows PET/CT images of ⁶⁸Ga-ZC-1 and ⁶⁸Ga-FAPI-04 in U87MG tumor mice at 0.5, 1, and 2 h in Test example 4.
Fig. 5 shows distribution statistics chart of ⁶⁸Ga-ZC-1 in U87MG tumor mice in Test example 4.
Fig. 6 shows areas under the curve (AUC) of time-tumor uptake for ⁶⁸Ga-ZC-1 and ⁶⁸Ga-FAPI-04 in Test example 4.

### DETAILED EMBODIMENTS

The compounds of the present invention, their preparation methods, and applications will be further described in detail below with reference to specific embodiments. It should be understood that the following examples are merely illustrative and explanatory of the present invention and should not be construed as limiting the protection scope of the present invention. All technologies implemented based on the above content of the present invention are covered within the protection scope of the present invention. Unless otherwise stated, the raw materials and reagents used in the following examples are commercially available products or may be prepared by known methods.

### Experimental Instruments and Materials

Animal Model: 5-7 week old female BALB/c mice (wt. 15-20 g) were purchased from the Shanghai Laboratory Animal Center, Chinese Academy of Sciences. The mice were housed under specific pathogen-free conditions. The housing environment was 25°C, humidity 35-45%, with 12-hour light-dark alternation. All animals had free access to water and food.

Cell line: U-87MG (Human Malignant Glioblastoma Cells), obtained from the Shanghai Institute of Materia Medica, Chinese Academy of Sciences.

Chemical reagents: acetonitrile, dimethyl sulfoxide (DMSO), N,N-diisopropylethylamine (DIPEA), dichloromethane (DCM), and N,N-dimethylformamide (DMF) were purchased from Shanghai Aladdin Biochemical Technology Co., Ltd. Trifluoroacetic acid (TFA) and N,N,N',N'-tetramethyl-o-(7-azabenzotriazol-1-yl)uronium hexafluorophospate (HATU) were purchased from Saen Chemical Technology (Shanghai) Co., Ltd. (S)-1-(2-aminoacetyl)-4,4-difluoropyrrolidine-2-carboxynitrile hydrochloride was purchased from Shaoyuan Technology (Shanghai) Co., Ltd. DOTA-NHS, 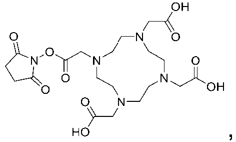 was purchased from J&K Scientific. Boc-gly-pro-OH, 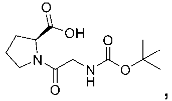 was purchased from Bide Pharmatech Co., Ltd. ICG-NHS, 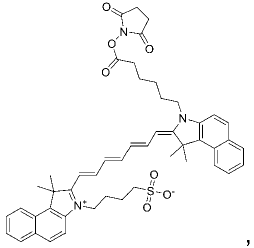 was purchased from J&K Scientific. FAPI-04 (CAS No.: 2374782-02-0), 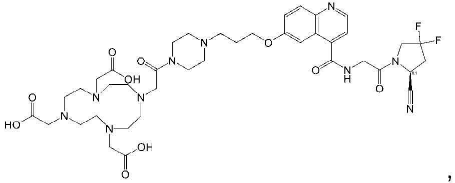 was purchased from Wuxi Jiehua Pharmaceutical Technology Co., Ltd. Biological reagents: DMEM medium, RPMI 1640 medium, fetal bovine serum, trypsin, phosphate-buffered saline (PBS), skim milk powder, 4% paraformaldehyde, and CCK8 kit were all purchased from Dalian Meilun Biotechnology Co., Ltd.

### Example 1: Preparation of compound ZC-1:

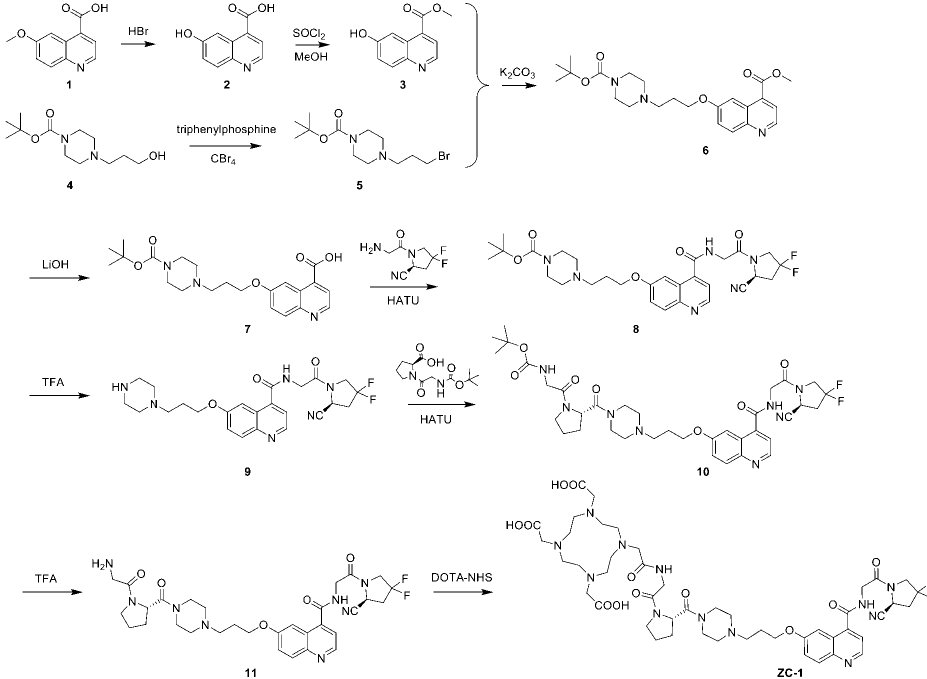

### Step 1: Synthesis of compound 2

Compound 1 (2.00 g, 24.61 mmol) was dissolved in 100 mL of an aqueous hydrogen bromide solution and stirred at 120 °C for 24 hours. The reaction solution was then alkalized to pH 6 with a 50% aqueous sodium hydroxide solution, to precipitate a large amount of a solid. The solid was collected and dried to obtain compound **2.** ¹H NMR (600 MHz, DMSO-*d*₆) δ 13.64 (s, 1H), 10.31 (s, 1H), 8.81 (d, *J* = 4.4 Hz, 1H), 8.11 (d, *J* = 2.8 Hz, 1H), 7.99 (d, *J* = 9.1 Hz, 1H), 7.88 (d, *J* = 4.4 Hz, 1H), 7.41 (dd, *J* = 9.1, 2.8 Hz, 1H). ¹³C NMR (126 MHz, DMSO-*d*₆) δ 168.26, 157.36, 147.00, 144.61, 133.83, 131.71, 126.68, 122.80, 122.73, 106.95. LR-ESI-MS (ESI)⁺: *m*/*z* calcd for C₁₀H₈NO₃⁺ [M+H]⁺: 190.1, found 190.2.

### Step 2: Synthesis of Compound 3

Compound 2 (4.1 g, 21.67 mmol) obtained in Step 1 was dissolved in 150 mL of MeOH. 5 mL of thionyl chloride was slowly added dropwise under an ice bath. After the addition was complete, the mixture was stirred overnight at 60 °C, and then the solvent was evaporated. The residue was precipitated with diethyl ether, stirred at 0 °C for 30 min, and the solid was collected by filtration to obtain compound 3. ¹H NMR (500 MHz, Methanol-*d*₄) δ 8.85 (d, *J* = 5.0 Hz, 1H), 8.18 (d, *J* = 2.7 Hz, 1H), 8.11 (d, *J* = 4.9 Hz, 1H), 8.05 (d, *J* = 9.2 Hz, 1H), 7.55 (dd, *J =* 9.2, 2.7 Hz, 1H), 4.07 (s, 3H). ¹³C NMR (126 MHz, Methanol-*d*₄) δ 165.09, 158.07, 143.12, 139.90, 135.66, 127.07, 126.76, 123.86, 122.18, 106.22, 51.59. LR-ESI-MS (ESI)⁻: *m*/*z* calcd for C₁₁H₈NO₃⁻ [M-H]⁻: 202.1, found. 202.5

### Step 3: Synthesis of Compound 5

Triphenylphosphine (5.9 g, 22.51 mmol, 1.1 eq) and carbon tetrabromide (7.46 g, 22.51 mmol, 1.1 eq) were added to a tetrahydrofuran solution (120 mL) of compound 4 (5 g, 20.46 mmol), and the reaction mixture was stirred at 25 °C for 12 h. After the reaction was complete, the reaction mixture was concentrated, and extracted with ethyl acetate. After washed with water, the combined organic phases were concentrated and purified by silica gel column chromatography (PE/EA = 3:1) to obtain compound **5.** ¹H NMR (400 MHz, Chloroform-*d*) δ 3.46 (dt, *J* = 15.6, 5.8 Hz, 6H), 2.57 - 2.31 (m, 6H), 2.10 - 2.00 (m, 2H), 1.47 (s, 9H).

### Step 4: Synthesis of Compound 6

Compound 3 (4 g, 19.69 mmol), compound 5 (6.05 g, 19.69 mmol), and potassium carbonate (5.44 g, 39.37 mmol) were sequentially added to 50 mL of N,N-dimethylformamide in a 100 mL flask. The system was heated to 60 °C and stirred overnight at 60 °C. The solvent was removed by vacuum distillation to obtain a crude product, which was purified by silica gel column chromatography (PE/EA = 1:1) to obtain compound 6. ¹H NMR (600 MHz, Chloroform-d) δ 8.84 (d, *J* = 4.5 Hz, 1H), 8.23 (d, *J =* 2.8 Hz, 1H), 8.05 (d, *J = 9.2* Hz, 1H), 7.92 (d, *J =* 4.5 Hz, 1H), 7.41 (dd, *J* = 9.2, 2.8 Hz, 1H), 4.20 (t, *J* = 6.2 Hz, 2H), 4.03 (s, 3H), 3.46 (t, *J* = 5.1 Hz, 4H), 2.64 - 2.41 (m, 6H), 2.07 (m, 2H), 1.47 (s, 9H). ¹³C NMR (151 MHz, Chloroform-d) δ 166.80, 158.60, 154.78, 146.89, 145.57, 132.35, 131.38, 126.78, 122.98, 122.78, 103.87, 79.67, 66.40, 55.18, 52.61, 28.44, 26.49. LR-ESI-MS (ESI)⁺: *m*/*z* calcd for C₂₃H₃₂N₃O₅⁺ [M+H]⁺: 430.2, found. 430.2.

### Step 5: Synthesis of Compound 7

100 mL of an aqueous solution of lithium hydroxide (167.26 mg, 6.98 mmol, 3 eq.) was added to a solution of compound 6 (1.00 g, 2.33 mmol, 1 eq.) in 100 mL of methanol and 50 mL of tetrahydrofuran. The mixture was stirred at 25 °C for 1 hour, and then concentrated under vacuum. The residue was diluted with water, adjusted to pH 5 with hydrochloric acid and stirred for another 15 minutes, and the resulting solid was separated by filtration and dried under vacuum to quantitatively obtain compound **7**. ¹H NMR (500 MHz, DMSO-*d*₆) δ 8.87 (d, *J =* 4.5 Hz, 1H), 8.19 (d, *J =* 2.8 Hz, 1H), 8.04 (d, *J* = 9.2 Hz, 1H), 7.93 (d, *J* = 4.4 Hz, 1H), 7.50 (dd, *J* = 9.2, 2.8 Hz, 1H), 4.20 (t, *J* = 6.1 Hz, 2H), 3.46 (t, *J* = 5.1 Hz, 4H), 2.98 - 2.76 (m, 6H), 2.11 (m, 2H), 1.42 (s, 9H). ¹³C NMR (126 MHz, DMSO-*d*₆) δ 167.74, 157.39, 153.51, 147.61, 144.79, 134.23, 131.18, 125.90, 122.60, 122.06, 104.56, 79.30, 65.60, 53.74, 51.64, 27.98, 24.59. LR-ESI-MS (ESI)⁻: *m*/*z* calcd for C₂₂H₂₈N₃O₅⁻ [M-H]⁻: 414.2, found. 414.4.

### Step 6: Synthesis of Compound 8

Compound 7 (100 mg, 528.64 µmol, 1.00 eq.) and (S)-1-(2-aminoacetyl)-4,4-difluoropyrrolidine-2-carboxynitrile hydrochloride (220 mg, 528.64 µmol, 1.0 eq.) were dissolved in DMF, added with HATU (241 mg, 634.36 µmol, 1.2 eq.) and N,N-diisopropylethylamine (205 mg, 1.59 mmol, 3 eq.), and stirred at 25 °C for 12 hours. The reaction mixture was filtered and the filtrate was purified by preparative HPLC to obtain compound 8. ¹H NMR (500 MHz, Methanol-*d*₄) δ 8.98 (d, *J* = 5.0 Hz, 1H), 8.24 (d, *J* = 2.7 Hz, 1H), 8.14 (d, *J* = 9.3 Hz, 1H), 7.87 (d, *J* = 5.0 Hz, 1H), 7.71 (dd, *J* = 9.3, 2.7 Hz, 1H), 5.16 (dd, *J* = 9.4, 3.4 Hz, 1H), 4.46 (t, *J* = 6.0 Hz, 2H), 4.44 - 4.08 (m, 6H), 3.74 - 3.43 (m, 4H), 3.31 - 2.73 (m, 6H), 2.48 - 2.34 (m, 2H), 1.50 (s, 9H). LR-ESI-MS (ESI)⁺: *m*/*z* calcd for C₂₄H₂₉F₂N₆O₃⁺ [M+H]⁺: 487.3, found 487.5.

### Step 7: Synthesis of Compound 9

Crude compound 8 (100.00 mg, 170.46 µmol) was dissolved in a solution of TFA (10 mL) in DCM (10 mL), stirred at room temperature for 1 hour, concentrated by evaporation, and added with diethylether to precipitate a solid. The solid was collected and dried under vacuum to obtain compound **9,** which was used directly in the next step without further purification. ¹H NMR (500 MHz, D₂O) δ 8.96 (d, *J* = 5.5 Hz, 1H), 8.16 (d, *J* = 9.3 Hz, 1H), 8.03 (d, *J* = 5.5 Hz, 1H), 7.81 (d, *J* = 2.6 Hz, 1H), 7.77 (d, *J =* 9.3 Hz, 1H), 5.10 (dd, *J =* 8.9, 4.1 Hz, 1H), 4.40 - 4.29 (m, 4H), 4.29 - 4.02 (m, 2H), 3.82 - 3.47 (m, 10H), 2.99 - 2.82 (m, 2H), 2.38 - 2.28 (m, 2H). LR-ESI-MS (ESI)⁺: *m*/*z* calcd for C₂₉H₃₇F₂N₆O₅⁺ [M+H]⁺: 587.3, found 587.5. LR-ESI-MS (ESI)⁺: *m*/*z* calcd for C₂₄H₂₉F₂N₆O₃⁺ [M+H]⁺: 487.3, found 487.5.

### Step 8: Synthesis of Compound 10

Boc-gly-pro-OH (100 mg, 367.24 µmol, 1 eq) and Compound 9 (178.67 mg, 367.24 µmol, 1 eq) were dissolved in DMF, added with HATU (167.57 mg, 440.69 µmol, 1 eq.) and N,N-diisopropylethylamine (237.32 mg, 1.84 mmol, 5 eq.), and stirred at 25 °C for 12 hours. After the reaction was complete, the reaction mixture was concentrated and purified by preparative HPLC to obtain compound 10 as a white solid. ¹H NMR (500 MHz, Chloroform-d) δ 8.58 (d, *J =* 7.5 Hz, 1H), 8.17 (t, *J = 8.7* Hz, 1H), 8.12 (d, *J = 7.6* Hz, 1H), 7.72 (d, *J =* 7.4 Hz, 1H), 7.66 - 7.65 (m, 1H), 7.24 (dd, *J* = 7.5, 1.5 Hz, 1H), 6.50 (t, *J* = 8.4 Hz, 1H), 4.75 (t, *J* = 7.0 Hz, 1H), 4.55 (t, *J* = 7.0 Hz, 1H), 4.09 - 3.82 (m, 8H), 3.53 (m, 6H), 2.73 - 2.48 (m, 8H), 2.03 (m, 2H), 1.94 - 1.80 (m, 4H), 1.40 (s, 9H). LR-ESI-MS (ESI)⁺: *m*/*z* calcd for C₃₆H₄₇F₂N₈O₇⁺ [M+H]⁺: 741.35, found 741.5.

### Step 9: Synthesis of Compound 11

Crude compound 10 (103.78 mg, 161.98 µmol) was dissolved in a solution of TFA (10 mL) in DCM (10 mL), stirred at room temperature for 1 hour, concentrated by evaporation, and added with diethylether to precipitate a solid. The solid was collected and dried under vacuum to obtain compound **11,** which was used directly in the next step without further purification.

### Step 10: Synthesis of Compound ZC-1

Crude compound 11 (100.00 mg, 156.08 µmol, 1 eq) was dissolved in 10 mL DMF, added with DOTA-NHS (391.37 mg, 780.41 µmol, 5 eq) and DIPEA (201.73 mg, 1.56 mmol, 10 eq), stirred at room temperature for 12 hours, and concentrated by evaporation. The residue was purified by preparative HPLC to obtain compound ZC-1 as a white solid. ¹H NMR (600 MHz, D₂O) δ 8.94 (d, *J =* 5.3 Hz, 1H), 8.15 (d, *J* = 3.2 Hz, 1H), 8.00 (d, *J* = 5.0 Hz, 1H), 7.79 (d, *J* = 9.3, Hz, 1H), 7.75 (d, *J* = 9.3 Hz, 1H), 5.10 (dd, *J* = 8.9, 4.1 Hz, 1H), 4.34 - 3.94 (m, 9H), 3.85 - 2.79 (m, 38H), 2.32 (s, 2H), 1.98 (s, 2H), 1.81 (s, 2H). HRMS calcd. for C₄₇H₆₅F₂N₁₂O₁₂⁺ [M+H]⁺: 1027.4807, found 1027.4805.

### Example 2: Preparation of Compound ZC-2

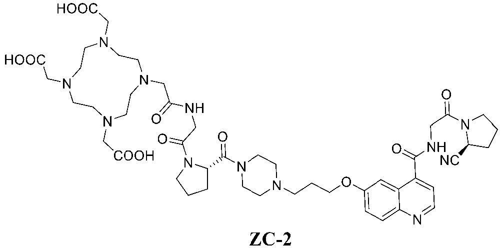

The preparation method of Compound ZC-2 was the same as that in Example 1, except that (S)-1-(2-aminoacetyl)-4,4-difluoropyrrolidine-2-carboxynitrile hydrochloride to be reacted with compound 7, was replaced with (S)-1-(2-aminoacetyl)pyrrolidine-2-carboxynitrile hydrochloride. Characterization data: ¹H NMR (400 MHz, Methanol-*d*₄) δ 8.59 (d, 1H), 8.19 (d, 1H), 7.75 - 7.70 (m, 1H), 7.61 (d, 1H), 7.28 (dd, 1H), 4.65 (m, 1H), 4.55 (m, 1H), 4.11 - 3.88 (m, 6H), 3.76 - 3.18 (m, 16H), 2.82 - 1.68 (m, 32H).

### Example 3: Preparation of Compound ZC-3

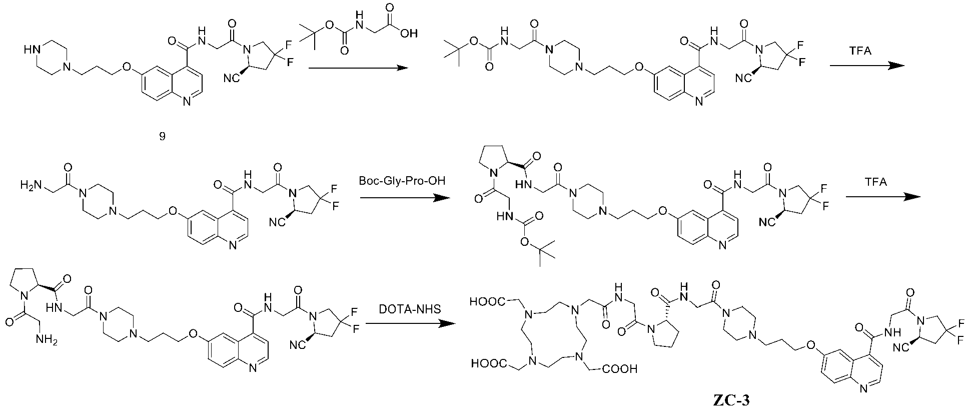

The preparation method of Compound ZC-3 was the same as that in Example 1, except that before step 8, compound 9 was reacted with BOC glycine. Specifically, compound 9 (1.0 eq) and BOC glycine (1.5 eq) were dissolved in DMF, added with 1.2 eq of HATU and 3 eq of DIPEA and stirred at room temperature for 8 hours. The resulting compound was deprotected in the presence of TFA and then reacted with Boc-gly-pro-OH. The resulting compound was again deprotected in the presence of TFA and then reacted with DOTA-NHS (reaction conditions were the same or similar to those in Example 1) to obtain ZC-3.

Characteristics data: ¹H NMR (400 MHz, Methanol-*d*₄) δ 8.59 (d, 1H), 8.19 (d, 1H), 7.75 - 7.69 (m, 1H), 7.61 (d, 1H), 7.28 (dd, 1H), 4.75 (m, 1H), 4.35 (m, 1H), 4.11 - 3.79 (m, 11H), 3.60 - 2.43 (m, 38H), 2.08 - 1.71 (m, 6H).

### Example 4: Preparation of Compound ZC-4

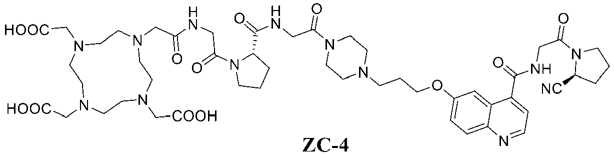

The preparation method of Compound ZC-4 was the same as that in Example 3, except that (S)-1-(2-aminoacetyl)-4,4-difluoropyrrolidine-2-carboxynitrile hydrochloride to be reacted with compound **7,** was replaced with (S)-1-(2-aminoacetyl)pyrrolidine-2-carboxynitrile hydrochloride.

Characterization data: ¹H NMR (400 MHz, Methanol-*d*₄) δ 8.59 (d, 1H), 8.19 (d, 1H), 7.73 - 7.70 (m, 1H), 7.61 (d, 1H), 7.28 (dd, 1H), 4.65 (m, 1H), 4.35 (m, 1H), 4.10 - 3.89 (m, 8H), 3.74 - 2.43 (m, 38H), 2.23 - 1.72 (m, 10H).

### Example 5: Preparation of Compound ZC-5

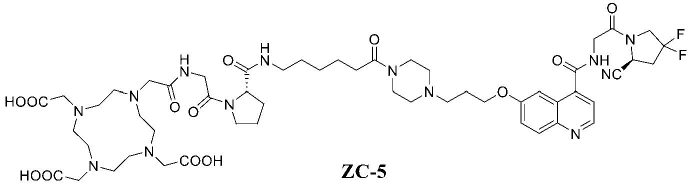

The preparation method of Compound ZC-5 was the same as that in Example 3, except that the BOC glycine to be reacted with compound **9** was replaced with 6-[(tert-butoxycarbonyl)amino]hexanoic acid.

Characterization data: ¹H NMR (400 MHz, Methanol-*d*₄) δ 8.59 (d, 1H), 8.19 (d, 1H), 7.74 - 7.69 (m, 1H), 7.61 (d, 1H), 7.28 (dd, 1H), 4.75 (m, 1H), 4.28 (m, 1H), 4.10 - 3.79 (m, 8H), 3.61 - 2.27 (m, 42H), 2.08 - 1.32 (m, 12H).

### Example 6: Preparation of Compound ZC-6

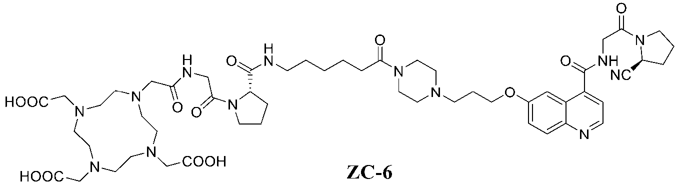

The preparation method of Compound ZC-6 was the same as that in Example 5, except that (S)-1-(2-aminoacetyl)-4,4-difluoropyrrolidine-2-carboxynitrile hydrochloride to be reacted with compound 7, was replaced with (S)-1-(2-aminoacetyl)pyrrolidine-2-carboxynitrile hydrochloride. Characterization data: ¹H NMR (400 MHz, Methanol-*d*₄) δ 8.59 (d, *J* = 7.5 Hz, 1H), 8.19 (d, 1H), 7.75 - 7.70 (m, 1H), 7.61 (d, 1H), 7.28 (dd, 1H), 4.65 (m, 1H), 4.28 m, 1H), 4.11 - 3.88 (m, 6H), 3.74 - 2.28 (m, 42H), 2.22 - 1.31 (m, 16H).

### Example 7: Preparation of Compound ZC-7

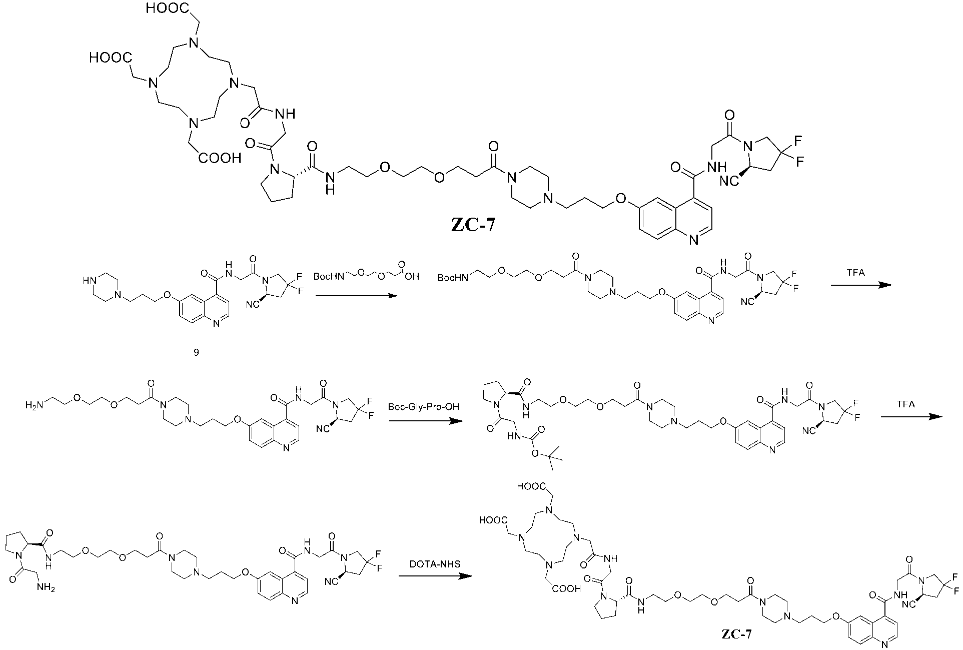

The preparation method of Compound ZC-7 was the same as that in Example 3, except that before step 8, compound **9** was reacted with N-tert-butoxycarbonyl-diethylene glycol-carboxylic acid. The specific reaction steps were as follows: compound **9** (1 eq) and N-tert-butoxycarbonyl-diethylene glycol-carboxylic acid (1.1 eq) were dissolved in DMF, added with 1.2 eq of HATU and 3 eq of DIPEA, and stirred at room temperature for 8 hours. The resulting compound was deprotected in the presence of TFA and then reacted with Boc-gly-pro-OH. The resulting compound was then deprotected in the presence of TFA and reacted with DOTA-NHS (reaction conditions were the same as or similar to those in Example 1) to obtain **ZC-7.**

Characterization data: ¹H NMR (400 MHz, Methanol-*d*₄) δ 8.59 (d, 1H), 8.19 (d, 1H), 7.74 - 7.69 (m, 1H), 7.61 (d, 1H), 7.28 (dd, 1.5 Hz, 1H), 4.75 (m, 1H), 4.34 (m, 1H), 4.12 - 3.77 (m, 10H), 3.73 - 2.39 (m, 48H), 2.10 - 1.65 (m, 6H).

### Example 8: Preparation of Compound ZC-8

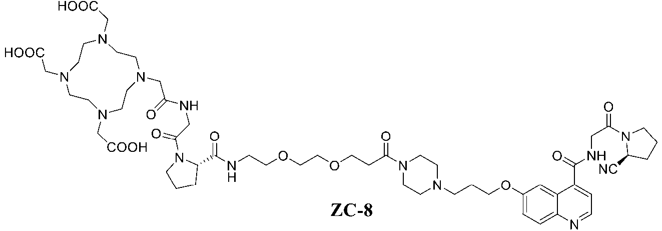

The preparation method of Compound ZC-8 was the same as that in Example 7, except that (S)-1-(2-aminoacetyl)-4,4-difluoropyrrolidine-2-carboxynitrile hydrochloride to be reacted with compound 7, was replaced with (S)-1-(2-aminoacetyl)pyrrolidine-2-carboxynitrile hydrochloride. Characterization data: ¹H NMR (400 MHz, Methanol-*d*₄) δ 8.59 (d, 1H), 8.19 (d, Hz, 1H), 7.75 - 7.67 (m, 1H), 7.61 (d, 1H), 7.28 (dd, 1.5 Hz, 1H), 4.65 (m, 1H), 4.34 (m, 1H), 4.12 - 2.40 (m, 56H), 2.23 - 1.71 (m, 10H).

### Example 9: Preparation of Compound ZC-9

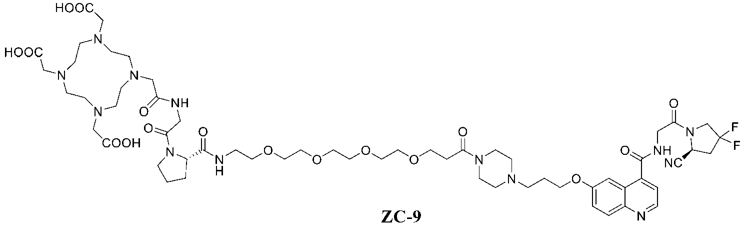

The preparation method of Compound ZC-9 was the same as that in Example 7, except that the N-tert-butoxycarbonyl-diethylene glycol-carboxylic acid to be reacted with compound **9** was replaced with 15-(Boc-amino)-4,7,10,13-tetraoxapentadecanoic acid.

Characterization data: ¹H NMR (400 MHz, Methanol-*d*₄) δ 8.59 (d, 1H), 8.19 (d, Hz, 1H), 7.79 - 7.68 (m, 1H), 7.61 (d, 1H), 7.28 (dd, 1.5 Hz, 1H), 4.75 (m, 1H), 4.34 (m, 1H), 4.12 - 2.36 (m, 66H), 2.08 - 1.70 (m, 6H).

### Example 10: Preparation of Compound ZC-10

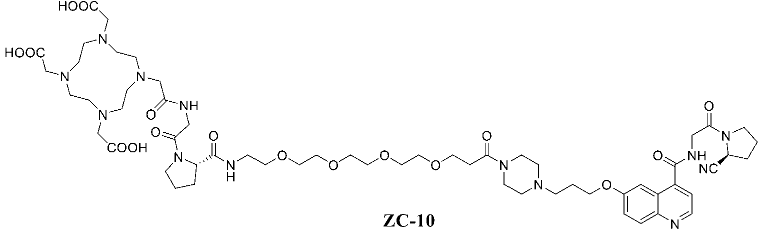

The preparation method of Compound ZC-10 was the same as that in Example 9, except that the (S)-1-(2-aminoacetyl)-4,4-difluoropyrrolidine-2-carboxynitrile hydrochloride to be reacted with compound 7 was replaced with (S)-1-(2-aminoacetyl)pyrrolidine-2-carboxynitrile hydrochloride. Characterization data: ¹H NMR (400 MHz, Methanol-*d*₄) δ 8.59 (d, 1H), 8.19 (d, Hz, 1H), 7.78 - 7.66 (m, 1H), 7.61 (d, 7.28 (dd, 1H), 4.65 (m, 1H), 4.34 (m, 1H), 4.14 - 2.40 (m, 64H), 2.25 - 1.69 (m, 10H).

### Example 11: Preparation of Compound ZC-11

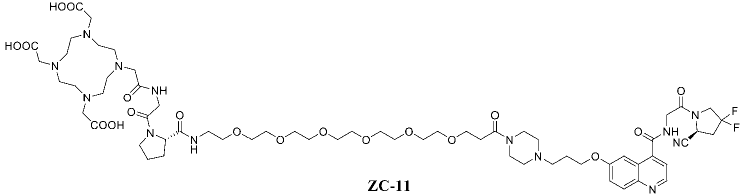

The preparation method of Compound ZC-11 was the same as that in Example 7, except that the N-tert-butoxycarbonyl-diethylene glycol-carboxylic acid to be reacted with compound **9** was replaced with 21-(BOC-amino)-4,7,10,13,16,19-hexaoxaheneicosanoic acid.

Characterization data: ¹H NMR (400 MHz, Methanol-*d*₄) δ 8.59 (d, 1H), 8.19 (d, 1H), 7.80 - 7.68 (m, 1H), 7.61 (d, 1H), 7.28 (dd, 1.5 Hz, 1H), 4.75 (m, 1H), 4.34 (m, 1H), 4.11 - 2.36 (m, 74H), 2.14 - 1.72 (m, 6H).

### Example 12: Preparation of Compound ZC-12

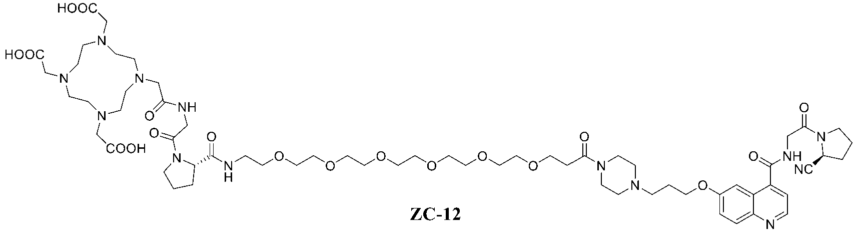

The preparation method of Compound ZC-12 was the same as that in Example 11, except that (S)-1-(2-aminoacetyl)-4,4-difluoropyrrolidine-2-carboxynitrile hydrochloride to be reacted with compound 7 was replaced with (S)-1-(2-aminoacetyl)pyrrolidine-2-carboxynitrile hydrochloride.

Characterization data: ¹H NMR (400 MHz, Methanol-*d*₄) δ 8.59 (d, 1H), 8.19 (d, 1H), 7.81 - 7.66 (m, 1H), 7.61 (d, 1H), 7.28 (dd, 1H), 4.65 (m, 1H), 4.34 (m, 1H), 4.14 - 2.40 (m, 72H), 2.23 - 1.70 (m, 10H).

### Example 13: Preparation of Compound ZC-13

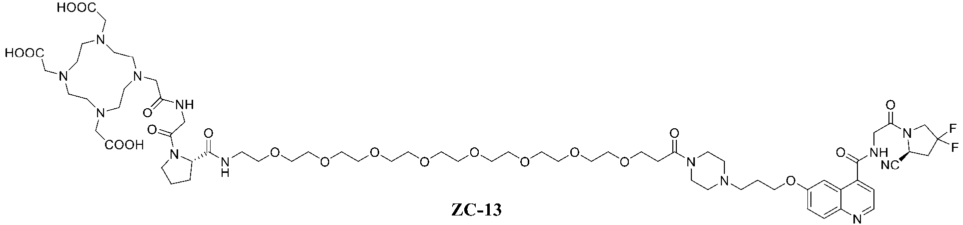

The preparation method of Compound ZC-13 was the same as that in Example 7, except that the N-tert-butoxycarbonyl-diethylene glycol-carboxylic acid to be reacted with compound **9** was replaced with N-tert-butoxycarbonyl-heptaethylene glycol-carboxylic acid.

Characterization data: ¹H NMR (400 MHz, Methanol-*d*₄) δ 8.59 (d, 1H), 8.19 (d, 1H), 7.78 - 7.69 (m, 1H), 7.61 (d, 1H), 7.28 (dd, 1H), 4.75 (m, 1H), 4.34 (m, 1H), 4.16 - 2.41 (m, 82H), 2.08 - 1.68 (m, 6H).

### Example 14: Preparation of Compound ZC-14

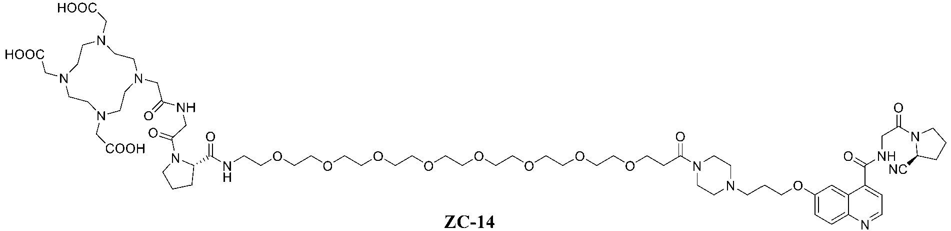

The preparation method of Compound ZC-14 was the same as that in Example 13, except that the (S)-1-(2-aminoacetyl)-4,4-difluoropyrrolidine-2-carboxynitrile hydrochloride to be reacted with compound 7 was replaced with (S)-1-(2-aminoacetyl)pyrrolidine-2-carboxynitrile hydrochloride.

Characterization data: ¹H NMR (400 MHz, Methanol-*d*₄) δ 8.59 (d, 1H), 8.19 (d, 1H), 7.78 - 7.67 (m, 1H), 7.61 (d, 1H), 7.28 (dd, 1H), 4.65 (m, 1H), 4.34 (m, 1H), 4.17 - 2.38 (m, 80H), 2.23 - 1.68 (m, 10H).

### Example 15: Preparation of compound ZC-ICG-1:

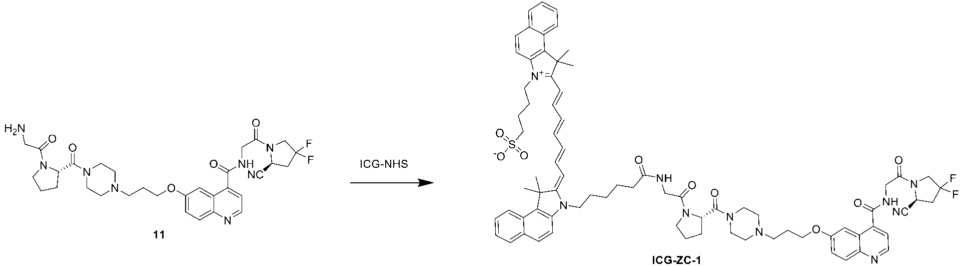

### Step 1: Synthesis of compound ZC-ICG-1

Crude compound 11 (100.00 mg, 156.08 µmol, 1 eq) was dissolved in 10 mL of DMF, and ICG-NHS (387 mg, 468.2 µmol, 3 eq) and DIPEA (201.73 mg, 1.56 mmol, 10 eq) were added. The mixture was stirred at room temperature for 12 hours, concentrated by evaporation, and purified by preparative HPLC to obtain compound **ZC-ICG-1** as a green solid. ¹H NMR (400 MHz, Methanol-*d*₄) δ 8.72 - 8.65 (m, 1H), 8.59 (d, 1H), 8.27 - 8.16 (m, 2H), 8.06 (d, 1H), 7.99 - 7.92 (m, 1H), 7.82 - 7.71 (m, 3H), 7.61 (d, 1H), 7.57 - 7.18 (m, 8H), 6.71 - 6.47 (m, 4H), 6.42 - 6.33 (m, 1H), 6.01 (d, 1H), 4.75 m, 1H), 4.52 (dt, 3H), 4.18 - 1.26 (m, 48H).

### Example 16: Preparation of ⁶⁸Ga-ZC-1

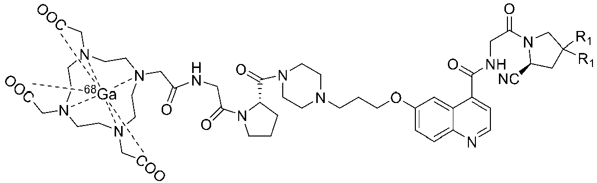

44 µL of ⁶⁸GaCl₃ was eluted from a ⁶⁸Ge-⁶⁸Ga generator with 0.1M hydrochloric acid, mixed with 0.5 µL of an aqueous solution of compound **ZC-1** with a concentration of 10 µg/µL. The mixture was adjusted pH to 4-5 by adding 400 µL of a sodium acetate solution, and reacted in a metal bath at 95°C for 10 minutes. After the reaction, the reaction mixture was diluted with 1 mL of physiological saline and injected into a C18 desalting column using a syringe. The C18 desalting column was then washed several times with 10 mL of physiological saline to remove ⁶⁸Ga³⁺ that was not chelated to **compound ZC-1** until the activity difference between two consecutive washes was less than 10 µCi. Finally, the C18 desalting column was eluted with 100-200 µL of ethanol to obtain **⁶⁸Ga-ZC-1.**

### Test Example 1: Docking Simulation Analysis of ZC-1, FAPI-04, and FAP

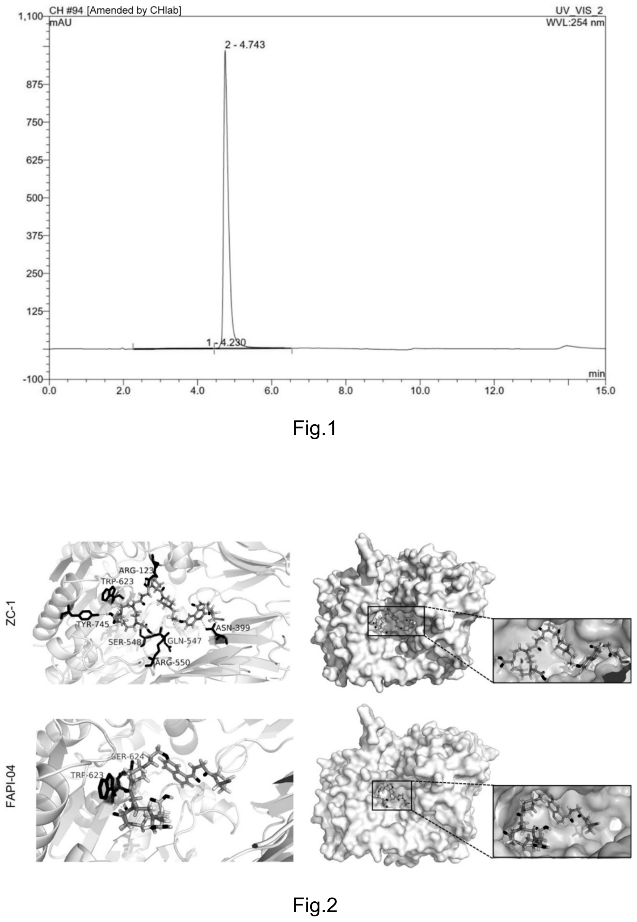

The three-dimensional structure of the FAP protein was downloaded from the RCSB protein database with a PDB ID of 1Z68. The homology model method was used to model compounds ZC-1 and FAPI-04. The docking simulation analysis results are shown in Fig. 2, where the red portion represents the interaction between FAP protein residues and small molecules, and potential hydrogen bonds are represented by yellow dashed lines. As shown in Fig. 2, the reference docking score for ZC-1 and FAP is 7.6177, while that for FAPI-04 is 6.3464. Compared to FAPI-04, ZC-1 could interact with Tyr⁷⁴⁵, Trp⁶²³, Arg¹²³, Ser⁵⁴⁸, Arg⁵⁵⁰, Gln⁵⁴⁷, and Asn³⁹⁹, while FAPI-04 could only interact with Trp⁶²³ and Ser⁶²⁴. Compared to FAPI-04, the Gly-Pro sequence of ZC-1 enhances the interaction between the compound and FAP, enabling it to bind to more amino acid residues of FAP protein, thus resulting in a stronger interaction between ZC-1 and FAP protein.

### Test Example 2: In Vitro FAP Enzyme Saturation Binding Assay of ⁶⁸Ga-ZC-1

1 × 10⁵ U87MG cells were seeded in each well of a 96-well cell plate and cultured for 24 hours. The solutions of ⁶⁸Ga-ZC-1 prepared in Example 16 and ⁶⁸Ga-labeled **FAPI-04** were diluted with culture medium to different concentrations of 1000 nM, 500 nM, 250 nM, 100 nM, 50 nM, 25 nM, and 0.78 nM. Meanwhile, FAPI-04 was diluted with complete culture medium to 100 µM to prepare an inhibitor solution. The upper layer of culture medium in the wells was aspirated from the plate and the cells were washed twice with 0.2 mL of PBS. The experiments were grouped as **⁶⁸Ga-ZC-1** or **⁶⁸Ga-FAPI-04** groups, each with a control group and an experimental group. The experimental group was added with 0.1 mL of blank culture medium, while the control group was added with 0.1 mL of 100 µM FAPI-04 solution. After incubation at 37°C for 30 minutes, both groups were added with 0.1 mL of the **⁶⁸Ga-ZC-1** or **⁶⁸Ga-FAPI-04** solution with different concentrations, respectively, and were incubated at 37°C for 2 hours. The solutions were then aspirated, and the cells were washed twice with PBS. Cells were lysed with 0.2 mL of 1 M NaOH solution and counted using a γ counter. All measurements were performed in duplicate. The results are shown in Table 1.

**Table 1. Kd values of ⁶⁸Ga-ZC-1 and ⁶⁸Ga-FAPI-04 binding to FAP protein**

| | K_{d} (nM) |
|---|---|
| ⁶⁸Ga-ZC-1 | 10.55 ± 0.57 |
| ⁶⁸Ga-FAPI-04 | 21.34 ± 0.81 |

Table 1 shows that compound **⁶⁸Ga-ZC-1** exhibited good FAP protein binding ability.

### Test Example 3: In vitro stability of ⁶⁸Ga-ZC-1 at 37°C

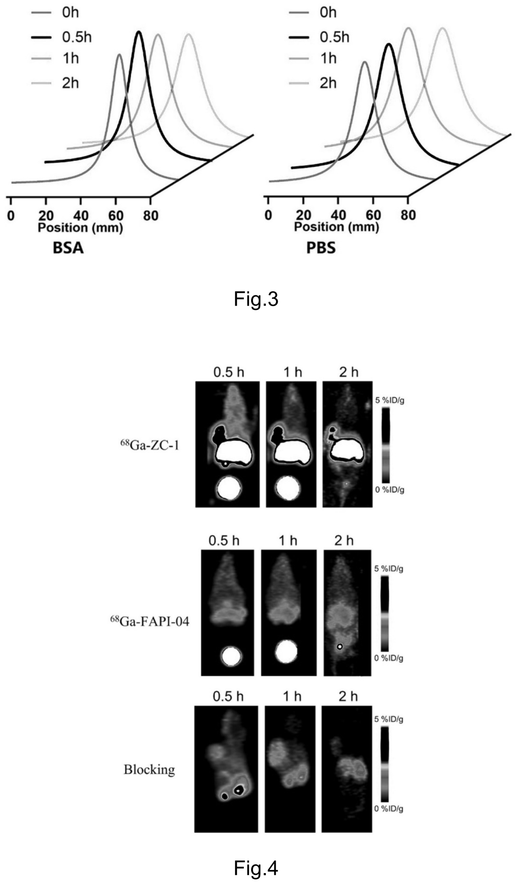

⁶⁸Ga-ZC-1 was dissolved in a FBS or BSA buffer at a concentration of 10 µg/µL. Then, 45 µL of a 1.5M sodium acetate solution and 430 µL of a gallium chloride eluent were added thereto and mixed thoroughly. After completion of labeling, the mixture was placed at 37°C for 0 min, 30 min, 60 min, and 120 min, respectively, and sampled for thin-layer chromatography, and the stability was calculated after detection with a γ-detector. The results are shown in Fig. 3.

Fig. 3 shows that ⁶⁸Ga-ZC-1 showed no significant demetallization in PBS and BSA buffers over two hours, indicating high stability, and thus it could be used for further experiments.

### Test Example 4: PET/CT Imaging of ⁶⁸Ga-ZC-1 and ⁶⁸Ga-FAPI-04 in U87MG Human Glioma-Bearing Mice

Experimental animals: U87MG human glioma-bearing mice; administration route: tail vein injection.

### Groups and Dosage:

⁶⁸Ga-ZC-1 Group: 150 µCi/200 µL of ⁶⁸Ga-ZC-1 administered via tail vein injection.

Block Group: Compound FAPI-04 (500-fold excess probe material) was administered via tail vein injection 30 minutes before tail vein injection of ⁶⁸Ga-ZC-1.

Control Group: 150 µCi/200 µL of 68Ga-FAPI-04 administered via tail vein injection.

PET/CT imaging was performed at 0.5, 1, and 2 h after ⁶⁸Ga-ZC-1 injection to observe the probe distribution in the mice and its enrichment in the tumor region. Results are shown in Figs. 4-6.

The results are shown in Fig. 4. ⁶⁸Ga-ZC-1 PET imaging of the U87MG xenograft model shows high tumor uptake and strong contrast between the tumor and background. Even 0.5 hours after injection, tumor accumulation rapidly reached 4.467 ± 0.379% ID/g, with signal gradually decreasing after 1 hour. Furthermore, ⁶⁸Ga-FAPI-04, as a clinically used agent anda contrast agent, showed significantly lower signal intensity in the tumor. Additionally, 0.5 hours after ⁶⁸Ga-ZC-1 injection, the U87MG tumor in the left anterior axilla of mice was clearly visible. Block experiments showed that pre-injection of non-radioactive FAPI-04 significantly reduced tumor uptake. This result indicates specific accumulation of ⁶⁸Ga-ZC-1 in vivo. In the control group, ⁶⁸Ga-FAPI-04 was visible in the tumor 0.5 hours after injection, but uptake was comparable to that in the liver; thereafter, uptake in the tumor area significantly decreased, and no signal was detected after 2 hours. Therefore, compared with the widely accepted ⁶⁸Ga-FAPI-04, ⁶⁸Ga-ZC-1 significantly enhances uptake in the tumor region and has a longer tumor retention time. The experimental results demonstrate that the ⁶⁸Ga-ZC-1 probe has the effect of in vivo tumor imaging.

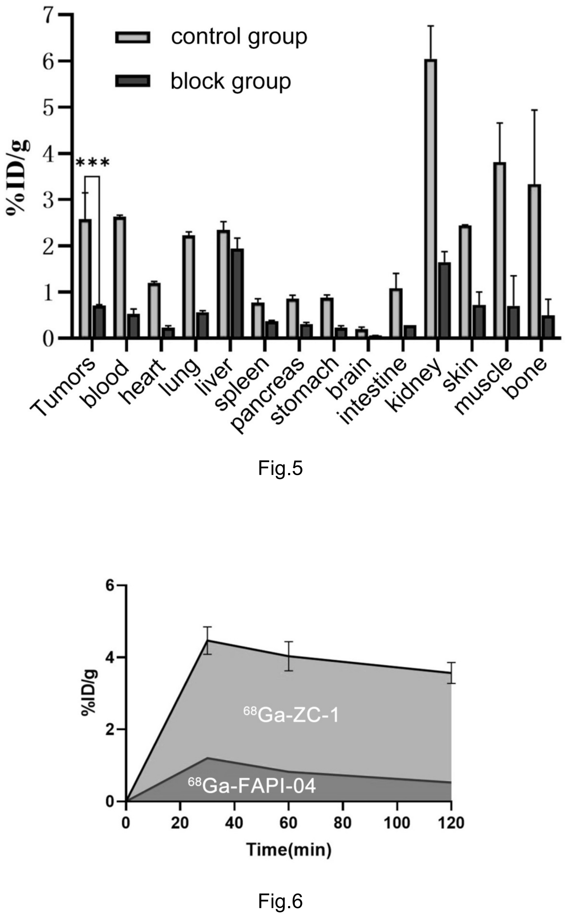

Fig. 5 shows the in vivo distribution statistics chart. The data in the chart show that after pre-injection of FAPI-04 as an inhibitor, the uptake of ⁶⁸Ga-ZC-1 significantly decreased in the tumor region after tail vein injection of ⁶⁸Ga-ZC-1, indicating that ⁶⁸Ga-ZC-1 could specifically target tumors with high FAP expression, and the tumor/brain distribution ratio was as high as 13. Fig. 6 shows the areas under the curve (AUC) of ⁶⁸Ga-ZC-1 and ⁶⁸Ga-FAPI-04 based on tumor uptake versus time, to compare the tumor uptakes of ⁶⁸Ga-ZC-1 and ⁶⁸Ga-FAPI-04 over time, which were 422.5 and 98.14, respectively, indicating that ⁶⁸Ga-ZC-1 has a longer tumor retention time.

## Claims

1. A compound of formula (I), or a pharmaceutically acceptable salt, enantiomer, diastereomer, racemate, transisomer, polymorph, solvate, or isotopically labeled compound thereof, 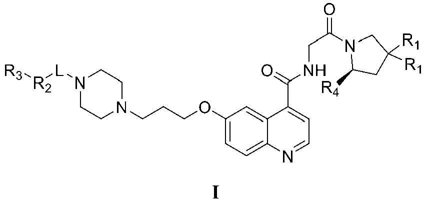
wherein, each of R₁s is independently halogen or H, preferably F;
R₂ is -(W₁)ₙ₁-(W₁)ₙ₂-(W₁)ₙ₃-(W₁)ₙ₄-(W₁)ₙ₅-(W₁)ₙ₆-, where n1 to n6 are each independently 0 or 1;
each of W₁s is independently selected from 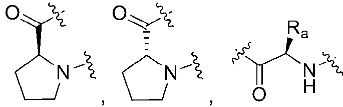 and 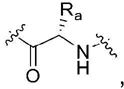 where Rₐ is selected from H, C1-C3 alkyl, the W₁s are connected from each other via an amide bond, and three or more adjacent W₁s are not simultaneously 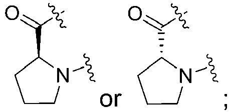
R₃ is a bifunctional chelator, a fluorescent reporter group, or a therapeutic drug;
L is -(CH₂)ₙ₇-, where n7 is an integer from 0 to 40; wherein each CH₂ is independently and optionally replaced by -O-, -NH-, -(CO)-, -NH(CO)-, or -(CO)-NH-, provided that no two adjacent CH₂ groups are replaced simultaneously;
R₄ is cyano (-CN) or borate (-B(OH)₂).

2. The compound of formula (I), or the pharmaceutically acceptable salt, enantiomer, diastereomer, racemate, transisomer, polymorph, solvate, or isotopically labeled compound thereof according to claim 1, wherein
the bifunctional chelator is selected from 1,4,7,10-tetraazacyclododecane-1,4,7,10-tetraacetic acid, triethylenetetramine, 2-(4,7-biscarboxymethyl[1,4,7]triazacyclononyl-1-yl-ethyl)carbonyl-methylamino] acetic acid, diethylenetriamine-N,N,N',N',N"-pentaacetic acid, N,N-di(2-hydroxyphenyl)ethylenediamine-N,N'-diacetic acid, and 2,2',",2‴-(5²,13²-dihydroxy-5⁵,13⁵-dimethyl-3,7,11,15-tetraaza-1,9(2,6)-dipyridin-5,13(1,3)-dibenzocyclohexanedione-3,7,1,11,15-tetrayl)tetraacetic acid; preferably 1,4,7,10-tetraazacyclododecane-1,4,7,10-tetraacetic acid; or
the fluorescent reporter group includes visible light groups, near-infrared region I groups, and near-infrared region II groups;
the visible light group may be any one selected from fluorescein, rhodamine, fluorescein isothiocyanate, cyanine fluorescent dyes (e.g., Cy2, etc.), green fluorescent protein, quantum dots, nanoparticles, F16, etc;
the near-infrared region I group may be any one selected from cyanine dyes (e.g., Cy3, Cy3.5, Cy5, Cy5.5, Cy7, Cy7.5), BODIPY dyes (e.g., fluoroboron dipyrrole, azafluoroboron dipyrrole), rhodamine dyes (e.g., rhodamine green, rhodamine 6G, tetramethylrhodamine, rhodamine B, lissamine rhodamine, X-rhodamine, Texas red, silyl rhodamine), quantum dots, nanoparticles, phthalocyanine;
the near-infrared region II group may be any one selected from cyanine dyes (e.g., Cy7, Cy7.5), D-A-D dyes (e.g., CH-1055, CH-4T, FT-TQT), BODIPY dyes (e.g., NJ960, NJ1030, NJ1060, PCP-BDP2), quantum dots, and nanoparticles; the therapeutic drug includes small molecule inhibitors, monoclonal antibody drugs, bioalkylating agents, cytotoxic drugs, hormonal drugs, and biological response modifiers; or
in formula (I),
each of the R₁s is H or F; and/or
R₂ is selected from 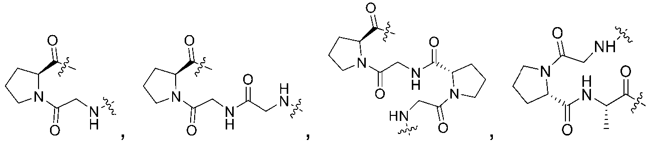 and 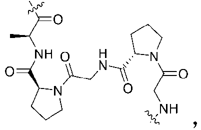 and preferably is 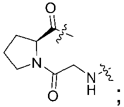 and/or
R₃ is selected from: 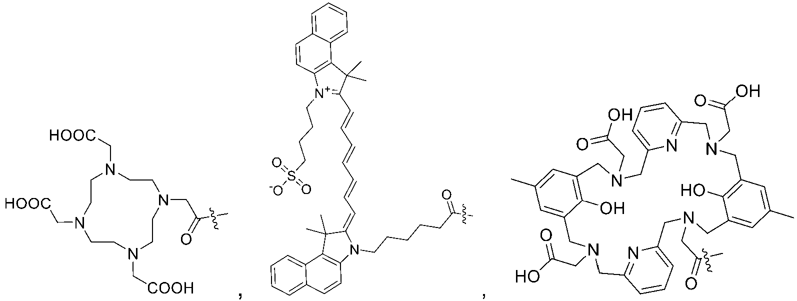 and 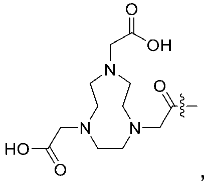 and preferably is 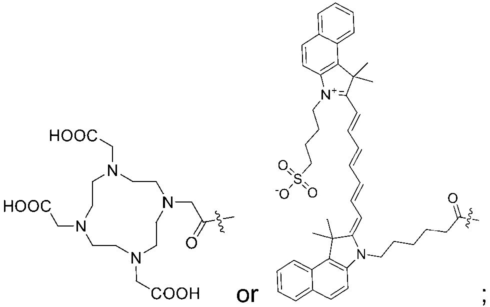 and/or
L is -(CH₂)ₙ₇-; n7 is an integer from 0 to 30, more preferably an integer from 0 to 12, and even more preferably is 0, 3, or 10; wherein each -CH₂- is independently and optionally replaced by -O-, -NH-, or -(CO)-, provided that no two adjacent -CH₂- groups are replaced; or
L is absent, or selected from -NH-(CH₂)ₙ₈-(CO)-, -NH-(CH₂CH₂O)ₙ₉-(CH₂)ₙ₈-(CO)-, where n8 and n9 are each independently an integer from 1 to 10 (e.g., 1, 2, 3, 4, 5, 6, 7, 8, 9, 10), preferably from 1 to 8, from 1 to 6, or from 1 to 4; and/or
R₄ is cyano (-CN).

3. The compound of formula (I), or the pharmaceutically acceptable salt, enantiomer, diastereomer, racemate, transisomer, polymorph, solvate, or isotopically labeled compound thereof according to claim 1 or 2, wherein the compound of formula (I) is selected from formulas (II-1) and (II-2): 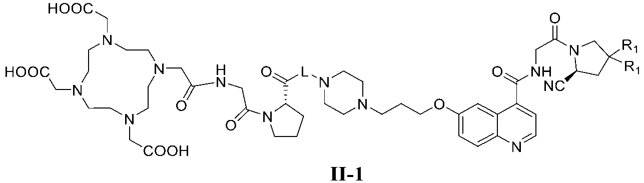 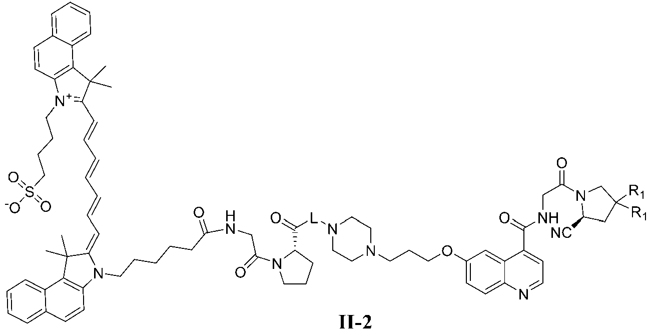 where R₁s and L are defined as those in the corresponding claims.

4. The compound of formula (I), or the pharmaceutically acceptable salt, enantiomer, diastereomer, racemate, transisomer, polymorph, solvate, or isotopically labeled compound thereof according to any one of claims 1 to 3, wherein the compound of formula (I) is selected from:

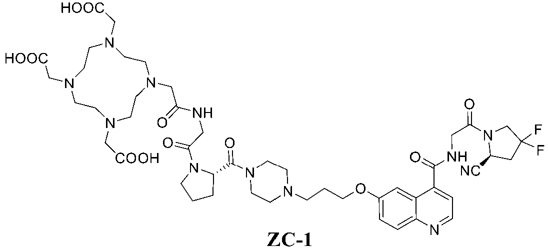

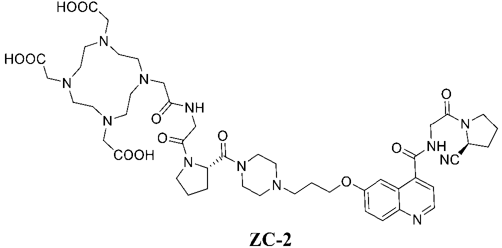

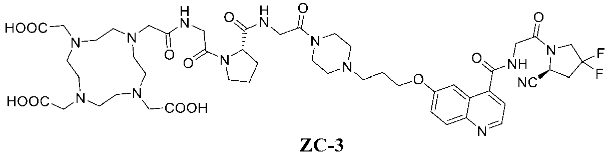

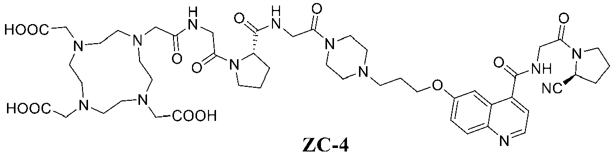

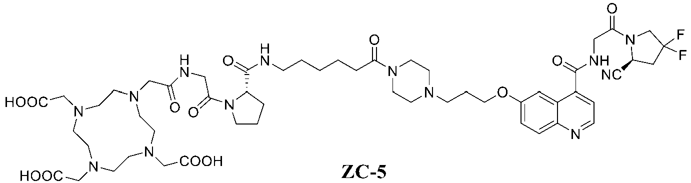

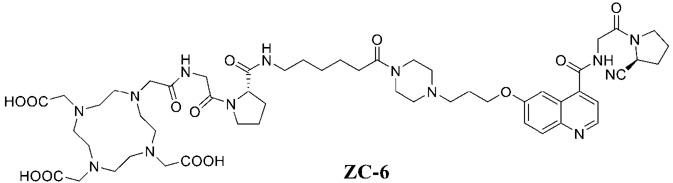

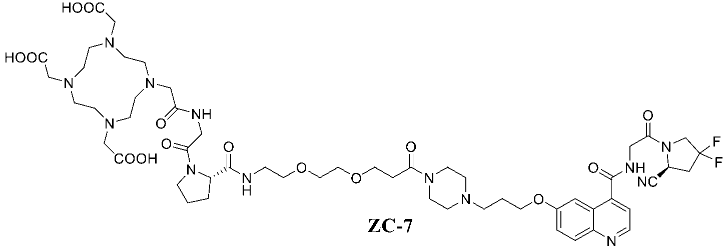

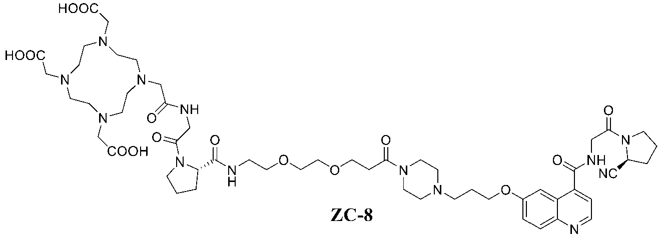

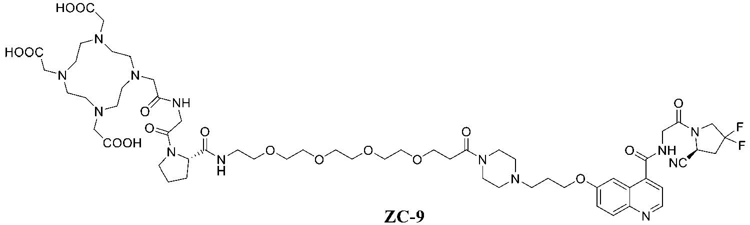

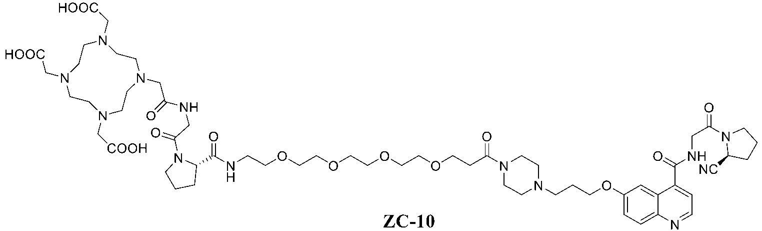

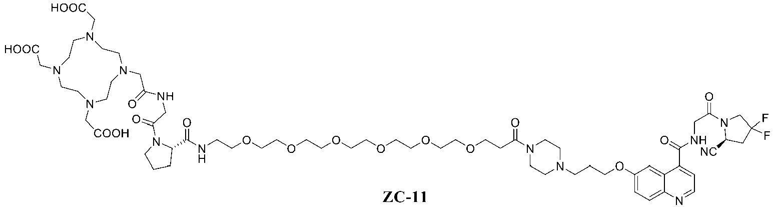

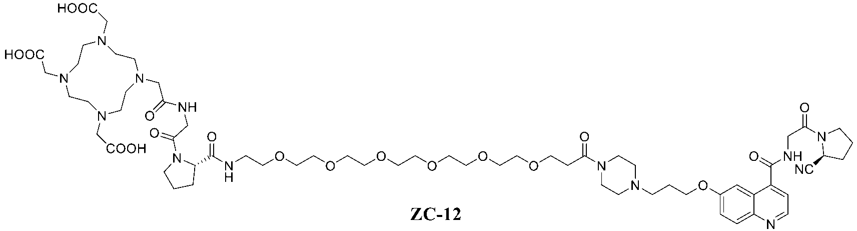

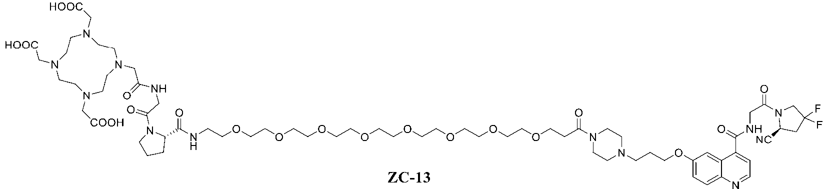

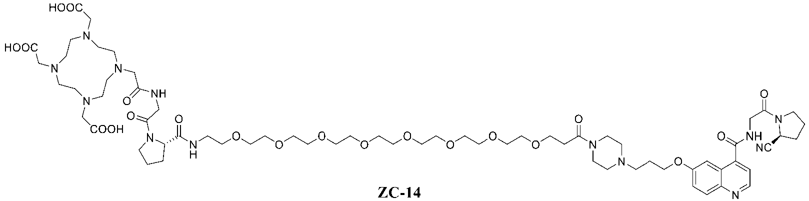

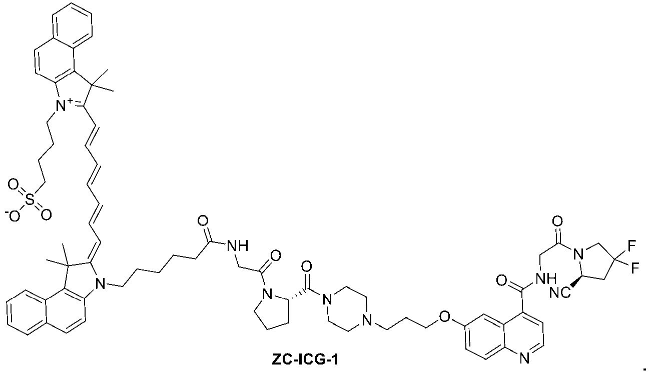

5. A method for preparing compound ZC-1, comprising the steps of: 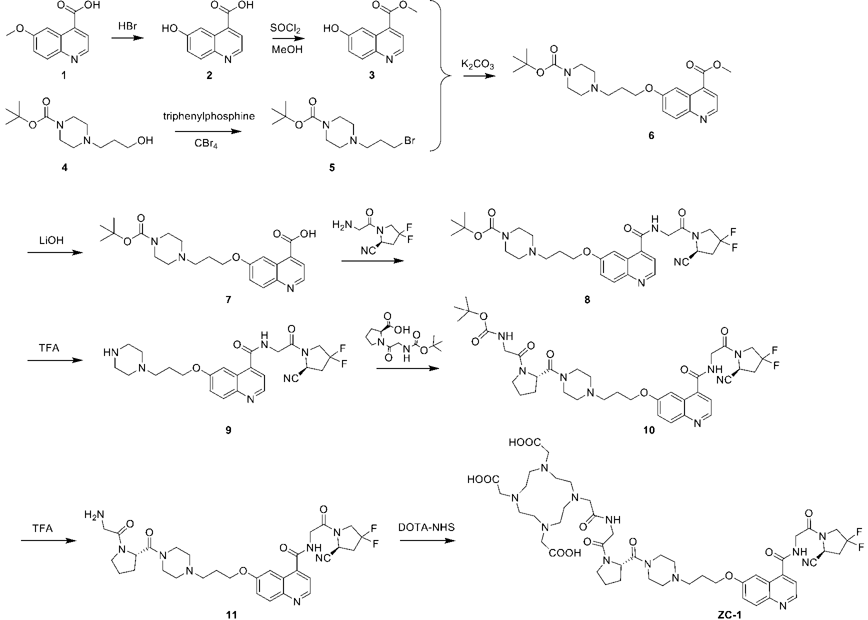
(1) demethylating compound 1 to obtain compound 2;
(2) esterifying compound 2 with methanol to obtain compound 3;
(3) brominating compound 4 to generate compound 5;
(4) substituting compound 5 with compound 3 to generate compound 6;
(5) ester-hydrolyzing compound 6 to generate compound 7;
(6) subjecting compound 7 to an amide condensation reaction with (S)-1-(2-aminoacetyl)-4,4-difluoropyrrolidine-2-carboxynitrile to generate compound 8;
(7) Boc-deprotecting compound 8 to generate compound 9, and then condensating compound 9 with Boc-gly-pro-OH to generate compound 10;
(8) Boc-deprotecting compound 10, and then reacting with DOTA-NHS to obtain compound ZC-1.

6. A radionuclide-labeled complex, which is obtained by labeling the compound of formula (I) or the pharmaceutically acceptable salt, enantiomer, diastereomer, racemate, transisomer, polymorph, solvate, or isotopically labeled compound thereof of any one of claims 1 to 4 as a ligand with a radionuclide M; particularly, the radionuclide M includes radiodiagnostic nuclides and radiotherapeutic nuclides;
particularly, the radiodiagnostic nuclide is any one selected from: ⁸⁶Y, ¹⁸F, ⁵¹Mn, ^{52m}Mn, ⁵²⁹Mn, Al[¹⁸F], ⁶⁴Cu, ⁶⁷Ga, ⁶⁸Ga, ⁸⁹Zr, 99mTc, ¹¹¹In, ¹²³I, ¹²⁴I, ¹²⁵I, ⁴⁴Sc and ⁴⁷Sc, preferably any one selected from: ⁸⁶Y, Al[¹⁸F], ⁶⁴Cu, ⁶⁸Ga, ⁸⁹Zr, ^{99m}Tc and ¹²⁴I;
particularly, the radiotherapeutic nuclide is any one selected from: ⁶⁷Cu, ⁹⁰Y, ¹²⁵I, ¹³¹I, ¹⁵³Sm, ¹⁶⁶Ho, ¹⁷⁷Lu, ¹⁸⁶Re, ¹⁸⁸Re, ²¹¹At, ²¹²Pb, ²⁰³Pb, ²¹²Bi, ²¹³Bi, ²²³Ra, ²²⁵Ac, ²²⁷Th; preferably any one selected from: ⁶⁷Cu, ⁹⁰Y, ¹²⁵I, ¹³¹I, ¹⁷⁷Lu, ²²³Ra, ²²⁵Ac, ²¹¹At,; more preferably ⁶⁸Ga, ¹⁷⁷Lu or ⁹⁰Y;
preferably, the radionuclide-labeled complex has a structure of formula (III): 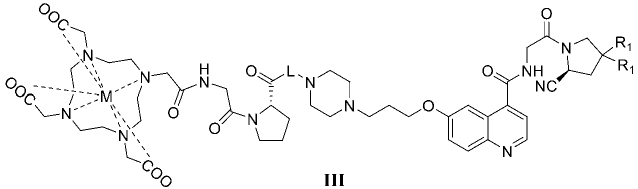
wherein,
L and Rₐs are defined as those in any one of claims 1 to 4;
M is defined as above; preferably, M is any one selected from ⁶⁸Ga, ¹⁷⁷Lu, and ⁹⁰Y;
more preferably, the radionuclide-labeled complex has the structure of:

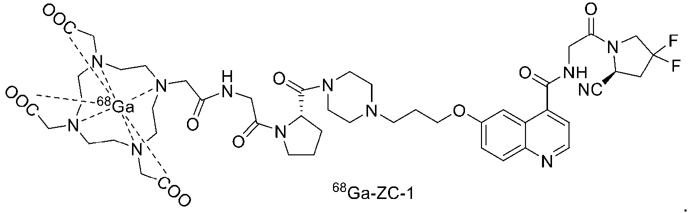

7. A method for preparing the radionuclide-labeled complex of claim 6, comprising labeling the compound, or the pharmaceutically acceptable salt, enantiomer, diastereomer, racemate, transisomer, polymorph, solvate, or isotopically labeled compound thereof of any one of claims 1 to 4 as a ligand with a radionuclide M;
particularly, the labeling method is a wet labeling method or a lyophilization labeling method;
particularly, the wet labeling method comprises the steps of: dissolving an appropriate amount of the compound of formula (I) or the pharmaceutically acceptable salt, enantiomer, diastereomer, racemate, transisomer, polymorph, solvate, or isotopically labeled compound thereof of any one of claims 1 to 4 in a buffer solution or deionized water to obtain a solution; adding a solution of the radionuclide M thereto and reacting under a sealed condition for 5-40 min to generate a radionuclide-labeled complex;
particularly, the lyophilization labeling method comprises the steps of: dissolving an appropriate amount of the compound of formula (I) or the pharmaceutically acceptable salt, enantiomer, diastereomer, racemate, transisomer, polymorph, solvate, or isotopically labeled compound thereof of any one of claims 1 to 4 in a buffer solution or deionized water to obtain a solution; after sterile filtration, dispensing the solution into a container, lyophilizing and sealing the container to obtain a lyophilized kit; adding an appropriate amount of an acetic acid solution or buffer solution to the lyophilized kit to dissolve the content, then adding a solution of the corresponding radionuclide M, and reacting under a sealed condition for 5-40 min to generate a radionuclide-labeled complex;
the radionuclide M is defined as that in claim 6.

8. A pharmaceutical composition comprising one or more selected from the compound, the pharmaceutically acceptable salt, enantiomer, diastereomer, racemic mixture, transisomer, polymorph, solvate, and isotopically labeled compound thereof of any one of claims 1 to 4, or the above radionuclide-labeled complex; and optionally a pharmaceutically acceptable excipient.

9. Use of the compound, or the pharmaceutically acceptable salt, enantiomer, diastereomer, racemic mixture, transisomer, polymorph, solvate, or isotopically labeled compound thereof of any one of claims 1 to 4, or the above radionuclide-labeled complex, in preparation of a reagent for inhibiting FAP activity.

10. Use of the compound,or the pharmaceutically acceptable salt, enantiomer, diastereomer, racemate, transisomer, polymorph, solvate, or isotopically labeled compound thereof of any one of claims 1 to 4, or the above radionuclide-labeled complex, in preparation of a medicament or reagent for diagnosis, prevention, and/or treatment of a disease **characterized by** high FAP expression; preferably, in preparation of a medicament or reagent for radionuclide therapy or imaging of a tumor with high FAP expression;
preferably, the tumor with high fap expression includes breast cancer, ovarian cancer, lung cancer, colorectal cancer, gastric cancer, and pancreatic cancer.
